# EUROPEAN PATENT APPLICATION

(11) **EP 2 253 636 A1**
(43) Date of publication of application: **24.11.2010**
(21) Application number: 09703992.9
(22) Date of filing: 23.01.2009
(51) Int. Cl.: C07D 493/10, A61K 31/343, A61P 25/00, A61P 43/00, C07D 307/77

(54) **NEURONAL CELL DEATH INHIBITOR**

(30) Priority: 23.01.2008 JP 2008012642
(71) Applicant: Lead Chemical Co. Ltd., Toyama-shi Toyama 930-0912 (JP)
(72) Inventor: NEMOTO, Hideo, Toyama-shi Toyama 939-8216 (JP); TOHDA, Chihiro, Imizu-shi Toyama 939-0351 (JP); MATSUYA, Yuji, Imizu-shi Toyama 939-0319 (JP)
(74) Representative: HOFFMANN EITLE
(86) International application number: PCT/JP2009/000253
(87) International publication number: WO 2009/093463

(57) **Abstract**

There is provided a neuronal cell death inhibitor having a high efficacy. A neuronal cell death inhibitor comprising a compound of general formula (1) below: 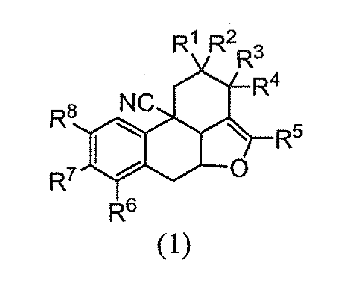 (where R¹ is a hydrogen atom or an optionally substituted hydroxy group; R² is a hydrogen atom; R³ is a hydrogen atom or an optionally substituted hydroxy group; R⁴ is a hydrogen atom or an optionally substituted hydroxy group, R³ together with R⁴ is optionally an oxo group or a group of -O-(CH₂)ₙ-O- (where n is an integer of 2 to 4), or R⁷ together with R⁴ optionally forms an unsaturated bond between carbon atoms, the respective carbon atoms being attached to R² and R⁴; R⁵ is a hydrogen atom or an optionally substituted lower alkyl group; each of R⁶, R⁷, and R⁸ is the same as or different from each other and is a hydrogen atom, an optionally substituted hydroxy group, an optionally substituted alkyl group, an optionally substituted aryl group, or an optionally substituted heteroaryl group); or a salt thereof.

## Description

### TECHNICAL FIELD

The present invention relates to a novel neuronal cell death inhibitor. The present invention specifically relates to a neuronal cell death inhibitor containing a compound having a tetracyclic dihydrofuran structure.

### BACKGROUND ART

A neuronal cell includes two neuritis, namely, a dendrite that receives information from other neuronal cells and an axon that sends information to other neuronal cells. Neuronal cell death or neurite atrophy caused by various factors inhibits normal signal transduction between neuronal cells to cause various diseases depending on the site of nervous system damage. Specific examples of the disease caused by a disorder of the central nervous system (brain and spinal cord) include Parkinson's disease, Alzheimer's disease, and amyotrophic lateral sclerosis, and specific examples of the disease caused by a disorder of the peripheral nervous system include multiple neuritis and Guillain-Barre syndrome. In particular, the central nervous system diseases are progressive, and effective treatment is not yet available. Neurotrophic factor-like agents have been studied as therapeutic agents for the central nervous system diseases. However, such agents mainly have neuroprotective action and do not clearly show neurite outgrowth effect in a neurodegenerative environment. In order to treat the central nervous system diseases, it is required that not only the neuronal cell death is inhibited but also the neurites of remaining neuronal cells are extended to normalize the signal transduction between the neuronal cells. Therefore, what is needed is the development of a therapeutic agent for central nervous system diseases having both the neuronal cell death inhibition effect and the neurite outgrowth effect.

In contrast, a tetracyclic dihydrofuran compound of general formula (1) below is known to have antiviral effect (Patent Document 1 and Non-patent Documents 1 to 3). However, it is completely unknown how the tetracyclic dihydrofuran compound of general formula (1) works on the neuronal cells.
Patent Document 1: Japanese Patent Application Publication No. JP-A-2002-255953.
Non-patent Document 1: Bioorg. Med. Chem. 2007,15,424-432.
Non-patent Document 2: Heterocycles 2004, 62, 207-211.
Non-patent Document 3: J. Org. Chem. 2004, 69, 7989-7993.

### DISCLOSURE OF THE INVENTION

### Problem to be Solved by the Invention

It is an object of the present invention to provide a novel neuronal cell death inhibitor and a prophylactic and therapeutic agent for neurological disorders.

### Means for Solving the Problem

The inventors of the present invention have carried out intensive studies in order to solve the above problems, and as a result, have found that the tetracyclic dihydrofuran compound of general formula (1) below extends the axons and dendrites and inhibits the neuronal cell death in cerebral cortical neurons of an Alzheimer's disease rat model treated with amyloid β (hereinafter called Aβ). This shows that the neurological disorders caused by the neuronal cell death or neurite atrophy can be treated by the tetracyclic dihydrofuran compound of general formula (1) below. From the above knowledge, the inventors of the present invention have accomplished the present invention.

Specifically, the present invention provides a neuronal cell death inhibitor including a compound of general formula (1) below:

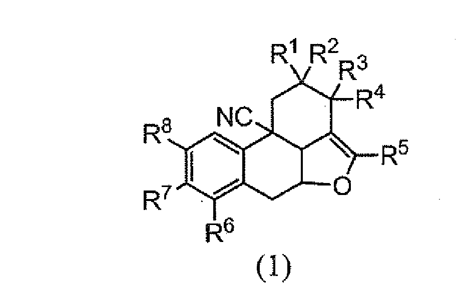

(where R¹ is a hydrogen atom or an optionally substituted hydroxy group; R² is a hydrogen atom; R³ is a hydrogen atom or an optionally substituted hydroxy group; R⁴ is a hydrogen atom or an optionally substituted hydroxy group, R³ together with R⁴ is optionally an oxo group or a group of -O-(CH₂)ₙ-O- (where n is an integer of 2 to 4), or R² together with R⁴ optionally forms an unsaturated bond between carbon atoms, the respective carbon atoms being attached to R² and R⁴; R⁵ is a hydrogen atom or an optionally substituted lower alkyl group; and each of R⁶, R⁷, and R⁸ is the same as or different from each other and is a hydrogen atom, an optionally substituted hydroxy group, an optionally substituted alkyl group, an optionally substituted aryl group, or an optionally substituted heteroaryl group)
or a salt thereof.

Furthermore, the present invention provides a therapeutic and/or prophylactic agent for a neurological disorder, and the agent includes a compound of general formula (1) or a salt thereof.
The present invention also provides a use of a compound of general formula (1) or a salt thereof for producing a neuronal cell death inhibitor.
The present invention also provides a use of a compound of general formula (1) or a salt thereof for producing a therapeutic and/or prophylactic agent for a neurological disorder.
The present invention also provides a method for inhibiting neuronal cell death characterized by including administering an effective amount of a compound of general formula (1) or a salt thereof.
The present invention also provides a method for treating and/or preventing a neurological disorder characterized by including administering an effective amount of a compound of general formula (1).

Among the compounds of general formula (1), a compound of general formula (1a) below is a novel compound. Accordingly, the present invention provides a compound of general formula (1a) below:

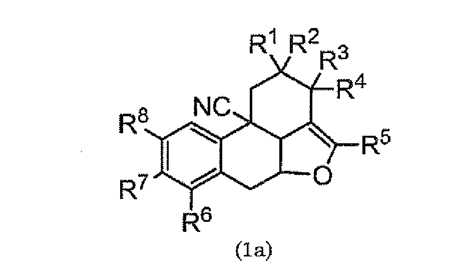

(where R¹ is a hydrogen atom or an optionally substituted hydroxy group; R² is a hydrogen atom; R³ together with R⁴ is a group of -O-(CH₂)ₙ-O- (where n is an integer of 2 to 4); R⁵ is a hydrogen atom or an optionally substituted lower alkyl group; and each of R⁶, R⁷, and R⁸ is the same as or different from each other and is a hydrogen atom, an optionally substituted hydroxy group, an optionally substituted alkyl group, an optionally substituted aryl group, or an optionally substituted heteroaryl group, unless each of R⁶ and R⁸ is a hydrogen atom and R⁷ is a hydrogen atom, an optionally substituted hydroxy group, an optionally substituted alkyl group, an optionally substituted aryl group, or an optionally substituted heteroaryl group)
or a salt thereof.

### Effects of the Invention

With the medical drug of the present invention, the neurites can be extended, the neuronal cell death can be remarkably inhibited, and the neurological disorders such as Alzheimer's disease can be treated.

### BRIEF DESCRIPTION OF THE DRAWINGS

[FIG. 1] FIG. 1 shows the neuronal cell death inhibition effect of Compounds 1 to 5 in rat cerebral cortical neurons treated with Aβ(25-35). Numbers in the figure represent the compound numbers, Cont represents a control group, and Veh represents a vehicle group. Hereinafter the same applies.
[FIG. 2] FIg. 2 shows the neuronal cell death inhibition effect of Compound 11 in rat cerebral cortical neurons treated with Aβ(25-35).
[FIG 3] FIG. 3 shows the dendrite outgrowth effect of Compounds 5 and 11 in rat cerebral cortical neurons treated with Aβ(25-35).
[FIG. 4] FIG. 4 shows the axon outgrowth effect of Compounds 8 and 11 in rat cerebral cortical neurons treated with Aβ(25-35).
[FIG. 5] FIG. 5 shows the dendrite outgrowth effect of Compounds 5, 11, 21, and 22 in rat cerebral cortical neurons treated with Aβ(1-42).
[FIG. 6] FIG. 6 shows the axon outgrowth effect of Compounds 5, 11, 21, and 22 in rat cerebral cortical neurons treated with Aβ(1-42).

### BEST MODES FOR CARRYING OUT THE INVENTION

In the present specification, the alkyl group means a C₁₋₂₀ alkyl group having a straight chain, a branched chain, a ring, or a combination thereof, and the lower alkyl group means an alkyl group having about 1 to 8 carbon atoms, preferably an alkyl group having about 1 to 6 carbon atoms having a straight chain, a branched chain, a ring, or a combination thereof. More specific examples of the lower alkyl group include a methyl group, an ethyl group, an n-propyl group, an isopropyl group, an n-butyl group, an isobutyl group, a tert-butyl group, an n-pentyl group, an isopentyl group, a tert-pentyl group, a hexyl group, a heptyl group, and an octyl group. Examples of the alkyl group include the lower alkyl group as well as a decyl group, an undecyl group, a dodecyl group, a tridecyl group, a tetradecyl group, a pentadecyl group, a hexadecyl group, a heptadecyl group, an octadecyl group, a nonadecyl group, and a didecyl group.

The substituent in "an optionally substituted hydroxy group" of R¹, R³, R⁴, R⁶, R⁷, and R⁸ in general formula (1) is not specifically limited, but examples thereof include an acyl group, a lower alkyl group, a sulfonyl group, and a silyl group. More specific examples thereof include acyl groups such as a formyl group, an alkanoyl group, a phenylcarbonyl group, an alkoxycarbonyl group, and a phenyl lower alkoxycarbonyl group, lower alkyl groups such as an allyl group, an alkoxy lower alkyl group, a phenyl lower alkyl group, and a tetrahydropyranyl group, sulfonyl groups such as a lower alkylsulfonyl group and an alkoxysulfonyl group, and silyl groups such as a lower alkylsilyl group and a phenylsilyl group.

Among the optionally substituted hydroxy groups, more preferable groups are a hydroxy group, a lower alkoxy group, a halogeno lower alkoxy group, a phenyl lower alkyloxy group, a halogenophenyl lower alkyloxy group, a lower alkylsulfonyloxy group, a halogeno lower alkylsulfonyloxy group, and a tri-lower alkylsilyloxy group. Examples of the lower alkoxy group include a methoxy group, an ethoxy group, a propoxy group, and an isopropyloxy group. Examples of the halogeno lower alkoxy group include a trifluoromethoxy group and a trichloromethoxy group. Examples of the phenyl lower alkyloxy group include a benzyloxy group and a phenethyloxy group. Examples of the halogenophenyl lower alkyloxy group include a fluorophenylmethoxy group, a difluorophenylmethoxy group, and a trifluorophenylmethoxy group. Examples of the lower alkylsulfonyloxy group include a methanesulfonyloxy group and an ethanesulfonyloxy group. Examples of the halogeno lower alkylsulfonyloxy group include a trifluoromethanesulfonyloxy group. Furthermore, examples of the tri(lower alkyl)silyloxy group include a trimethylsilyloxy group and a triisopropylsilyloxy group.

In "an optionally substituted lower alkyl group " of R⁵ and "an optionally substituted alkyl group", "an optionally substituted aryl group", and "an optionally substituted heteroaryl group," of each of R⁶, R⁷, and R⁸ in general formula (1), the substituent, the substitution site, and the number of the substituents are not specifically limited. Examples of the substituent include a hydroxy group, an alkoxy group (the alkyl moiety is the same as that in the alkyl group above), a carboxy group, a halogen atom (any of a fluorine atom, a chlorine atom, a bromine atom, or an iodine atom), an optionally substituted amino group (examples of the substituent include an alkyl group and an acyl group), an optionally substituted aryl group (examples of the substituent include an alkyl group, an acyl group, and a halogen atom), and an optionally substituted heteroaryl group (examples of the substituent include an alkyl group, an acyl group, and a halogen atom).

Specific examples of the optionally substituted lower alkyl group include a lower alkyl group, a hydroxy lower alkyl group, and a halogeno lower alkyl group. Specific examples of the optionally substituted aryl group include a phenyl group and a halogenophenyl group. Specific examples of the optionally substituted heteroaryl group include a pyridyl group and a furyl group.

In the compound of general formula (1), each of R¹ and R⁵ is preferably a hydrogen atom. In the compound of general formula (1), it is preferable that R² is a hydrogen atom and that R³ together with R⁴ is a group of -O-(CH₂)ₙ-O- (where n is an integer of 2 to 4), and it is more preferable that R² is a hydrogen atom and that R³ together with R⁴ is the group of -O-(CH₂)₂-O-.

In the compound of general formula (1), it is preferable that each of R⁶, R⁷, and R⁸ is the same as or different from each other and is a hydrogen atom; an optionally substituted hydroxy group; or an optionally substituted aryl group. Among them, it is more preferable that each of R⁶, R⁷, and R⁸ is the same as or different from each other and is a hydrogen atom; a hydroxy group optionally substituted with a sulfonyl group, a silyl group, or a lower alkyl group optionally substituted with a halogen atom; or an aryl group substituted with a halogen atom.

It is more preferable that each of R⁶, R⁷, and R⁸ is the same as or different from each other and is a hydrogen atom; a hydroxy group, a lower alkoxy group, a halogeno lower alkoxy group, or a tri(lower alkyl)silyloxy group, it is even more preferable that each of one or more of R⁶, R⁷, and R⁸ is a hydroxy group, a lower alkoxy group, a halogeno lower alkoxy group, or a tri-lower alkylsilyloxy group and that each of the remainder is a hydrogen atom, and it is yet even more preferable that each of one or more of R⁶, R⁷, and R⁸ is a hydroxy group or a lower alkoxy group and that each of the remainder is a hydrogen atom.

It is yet even more preferable that R⁶ is a hydrogen atom, that each of one or more of R⁷ and R⁸ is a hydroxy group or a lower alkoxy group, and that the remain is a hydrogen atom, and it is most preferable that R⁶ is a hydrogen atom, that R⁷ is a hydrogen atom, a hydroxy group, or a lower alkoxy group, and that R⁸ is a hydroxy group or a lower alkoxy group.

The compound of general formula (1) can be produced, for example, by the methods described in Japanese Patent Application Publication No. JP-A-2002-255953 and Bioorg. Med. Chem. 2007, 15, 424-432.

The compound of general formula (1) in which R⁸ is a hydroxy group can be produced according to Process A below.

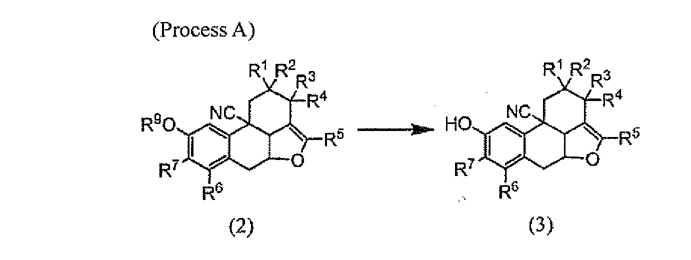

(where R⁹ is a protective group for the hydroxy group; and each of R¹ to R⁷ is the same as that in the above).

In Process A, a protective group of R⁹ for the hydroxy group in the compound of general formula (2) is deprotected to produce a compound of general formula (3).
A conventional protective group may be used for the protective group of R⁹ for the hydroxy group, and examples thereof include a benzyl group, an acetyl group, and an alkylsilyl group (such as a trimethylsilyl group, a tert-butyldimethylsilyl group, a triisopropylsilyl group, and a tert-butyldiphenylsilyl group).

The deprotection may be carried out in usual ways such as reduction, hydrolysis, acid treatment, and fluoride treatment depending on the type of the protective group to be removed.

In the case of the reduction, for example, the treatment may be carried out in an appropriate solvent (such as methanol and ethanol) in a hydrogen atmosphere using a catalyst (such as palladium-carbon and platinum).
In the case of the hydrolysis, the treatment may be carried out tin an appropriate solvent (such as tetrahydrofuran, dioxane, methanol, ethanol, and water) with a base (such as sodium hydroxide, potassium hydroxide, lithium hydroxide, sodium methoxide, and sodium ethoxide).

In the case of the acid treatment, the treatment may be carried out in an appropriate solvent (such as methanol and ethanol) with an acid (such as hydrochloric acid, p-toluenesulfonic acid, methanesulfonic acid, and trifluoroacetic acid).

In the case of the fluoride treatment, the treatment may be carried out in an appropriate solvent (such as methanol, ethanol, acetonitrile, and tetrahydrofuran) with a fluoride (such as hydrogen fluoride, hydrogen fluoride-pyridine, and tetrabutylammonium fluoride).

The reaction temperature varies depending on the deprotection method, the reaction reagent, and the like, but the reaction is usually carried out at -50°C to 100°C and preferably at 0°C to 50°C. The reaction time varies depending on the reaction temperature, the deprotection method, the reaction reagent, and the like, but the reaction is usually carried out for 30 minutes to 48 hours and preferably for 1 hour to 24 hours.
The compound of general formula (2) as the starting material in Process A can be produced by the method described in Bioorg. Med. Chem. 2007, 15, 424-432.

The compound of general formula (1) in which R⁷ is an optionally substituted aryl group can be produced according to Process B below.

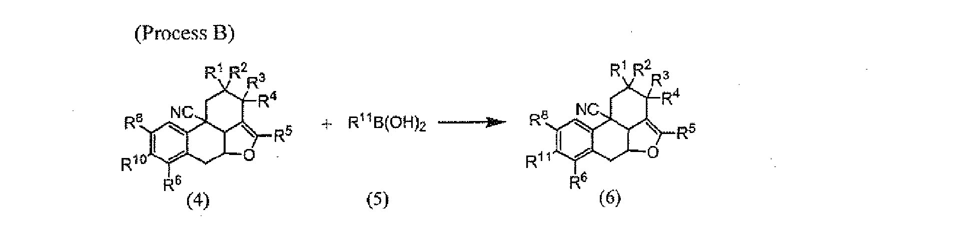

(where R¹⁰ is a halogen atom or a trifluoromethanesulfonyloxy group; R¹¹ is an optionally substituted aryl group; and each of R¹ to R⁶ and R⁸ is the same as that in the above).

In Process B, a compound of general formula (4) and boronic acid compounds of general formula (5) are subjected to Suzuki coupling reaction in the presence of a base and a palladium catalyst to produce a compound of general formula (6).

The solvent used in Process B is not specifically limited as long as the solvent does not inhibit the coupling reaction. Examples thereof include ethers such as diethyl ether, diisopropyl ether, tetrahydrofuran, dioxane, and 1,2-dimethoxyethane, alcohols such as methanol, ethanol, and propanol, hydrocarbons such as benzene, toluene, xylene, carbon disulfide, cyclohexane, and hexane, amides such as formamide, N,N-dimethylformamide, and N,N-dimethylacetamide, halogenated hydrocarbons such as dichloromethane, chloroform, carbon tetrachloride, and 1,2-dichloroethane, nitriles such as acetonitrile, propionitrile, and isobutyronitrile, sulfoxides such as dimethylsulfoxide, water, and a mixed solvent thereof.

Examples of the palladium catalyst used in Process B include tetrakis(triphenylphosphine)palladium, palladium acetate, palladium chloride, 1,1-bis(diphenylphosphino)ferrocenedichloropalladium, and dichlorobis(triphenylphosphine)palladium.

Examples of the base used in Process B include basic salts such as sodium carbonate, potassium carbonate, cesium carbonate, and sodium hydrogen carbonate, aromatic amines such as pyridine and lutidine, tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine.

The reaction temperature varies depending on the solvent, the catalyst, the base, and the like, but the reaction is usually carried out at 0°C to 250°C and preferably at 50°C to 150°C. The reaction time varies depending on the reaction temperature, the solvent, the base, and the like, but the reaction is usually carried out for 5 minutes to 48 hours and preferably for 30 minutes to 24 hours.

The compound of general formula (4) as the starting material in Process B can be produced by a conventionally known production method, for example, by triflation of the compound of general formula (1) in which R⁷ is a hydroxy group described in J. Org. Chem. 2004, 69, 7989-7993 using trifluoromethanesulfonic anhydride and the like.

The compound of general formula (1) produced by each of the above processes can be purified and isolated by a conventionally known separation and purification technique such as concentration, solvent extraction, filtration, recrystallization, and various chromatographic methods.

The compound of general formula (1) may form a salt. The salt type is not specifically limited, but generally, when a basic group such as an amino group is included in the molecule, an acid addition salt is formed. In contrast, when an acidic group such as a carboxy group and a phenolic hydroxy group is included in the molecule, a base addition salt is formed. Examples of the acid addition salt include a mineral acid salt such as a hydrochloride, a sulfate, and a nitrate and an organic acid salt such as a p-toluenesulfonate, a methanesulfonate, an oxalate, a fumarate, a maleate, and an acetate. Furthermore, examples of the base addition salt include metal salts such as a sodium salt, a potassium salt, a calcium salt, and a magnesium salt, an ammonium salt, and organic amine salts such as a methylamine salt and an ethylamine salt. An amino acid addition salt such as a glycine salt may also be formed.

The compound of general formula (1) has three asymmetric carbons in the ring and may have further one or more asymmetric carbons depending on the type of a substituent. Each of the asymmetric carbons may have any of an R or S configuration. In the range of the compound of general formula (1), stereoisomers such as optically active compounds and diastereoisomers based on the asymmetric carbons, any mixture of the stereoisomers, and racemates are included. Furthermore, the range of the compound of general formula (1) includes the compound of general formula (1) and a salt thereof as well as any hydrates and solvates thereof.

Among the compounds of general formula (1), the compounds of (1) to (16) described below are exemplified as specifically preferred compound.
(1) 5a,10c-dihydro-1H,6H-5-oxa-8-(trifluoromethoxy)acephenanthrylene-10b-carbonitrile (hereinafter called Compound 1)
(2) 8-trifluoromethoxy-2,3,5a,10c-tetrahydro-1H,6H-5-oxa-3-oxo-acephenanthrylene-10b-carbonitrile ethylene ketal (hereinafter called Compound 2)
(3) 8-triisopropylsilyloxy-2,3,5a,10c-tetrahydro-1H,6H-5-oxa-3-oxo-acephenanthrylene-10b-carbonitrile ethylene ketal (hereinafter called Compound 3)
(4) 8-methoxy-2,3,5a,10c-tetrahydro-1H,6H-5-oxa-3-oxo-acephenanthrylene-10b-carbonitrile ethylene ketal (hereinafter called Compound 4)
(5) 8-hydroxy-2,3,5a,10c-tetrahydro-1H,6H-5-oxa-3-oxo-acephenanthrylene-10b-carbanitrile ethylene ketal (hereinafter called Compound 5)
(6) 8-trifluaromethanesulfonyloxy-2,3,5a,10c-tetrahydro-1H,6H-5-oxa-3-oxo-acephenanthrylene-10b-carbonitrile ethylene ketal (hereinafter called Compound 6)
(7) 8-benzyloxy-2,3,5a,10c-tetrahydro-1H,6H-5-oxa-3-oxo-acephcnanthrylene-10b-carbonitrile ethylene ketal (hereinafter called Compound 7)
(8) 8-(3,5-difluorobenzyloxy)-2,3,5a,10c-tetrahydro-1H,6H-5-oxa-3-oxo-acephenanthrylene-10b-carbonitrile ethylene ketal (hereinafter called Compound 8)
(9) 8-phenyl-2,3,5a,10c-tetrahydro-1H,6H-5-oxa-3-oxo-acephenanthrylene-10b-carbonitrile ethylene ketal (hereinafter called Compound 9)
(10) 8-(2,4,6-trifluorophenyl)-2,3,5a,10c-tetrahydro-1H,6H-5-oxa-3-oxo-acephenanthrylene-10b-carbonitrile ethylene ketal (hereinafter called Compound 10)
(11) 9-hydroxy-2,3,5a,10c-tetrahydro-1H,6H-5-oxa-3-oxo-acephenanthrylene-10b-carbonitrile ethylene ketal (hereinafter called Compound 11)
(12) 7-hydroxy-2,3,5a,10c-tetrahydro-1H,6H-5-oxa-3-oxo-acephenanthrylene-10b-carbonitrile ethylene ketal (hereinafter called Compound 12)
(13) 8,9-dihydroxy-2,3,5a,10c-tetrahydro-1H,6H-5-oxa-3-oxo-acephenanthrylene-10b-carbanitrile ethylene ketal (hereinafter called Compound 13)
(14) 7,9-dihydroxy-2,3,5a,10c-tetrahydro-1H,6H-5-oxa-3-oxo-acephenanthrylene-10b-carbonitrile ethylene ketal (hereinafter called Compound 14)
(15) 7,8,9-trihydroxy-2,3,5a,10c-tetrahydro-1H,6H-5-oxa-3-oxo-acephenanthrylene-10b-carbonitrile ethylene ketal (hereinafter called Compound 15)
(16) 2,3,5a,10c-tetrahydro-1H,6H-5-oxa-3-oxo-acephenanthrylene-10b-carbonitrile ethylene ketal (hereinafter called Compound 16)
(17) 8-hydroxy-9-methoxy-2,3,5a,10c-tetrahydro-1H,6H-5-oxa-3-oxo-acephenanthrylene-10b-carbonitrile ethylene ketal (hereinafter called Compound 21)
(18) 9-hydroxy-8-methoxy-2,3,5a,10c-tetrahydro-1H,6H-5-oxa-3-oxo-acephenanthrylene-10b-carbonitrile ethylene ketal (hereinafter called Compound 22)

The prophylactic and/or therapeutic agent for the neurological disorder is desirably a compound that inhibits the neuronal cell death as well as extends the neurites of the remaining neuronal cells to normalize the signal transduction between the nerve cells. The compound of general formula (1) has the neuronal cell death inhibition effect and the neurite outgrowth effect and therefore is useful as an active component in a medical drug for preventing and/or treating the neurological disorders caused by the neuronal cell death or the neurite atrophy. Neurological disorders to be applied with the medical drug of the present invention are not specifically limited. Examples thereof include Alzheimer's disease, cerebrovascular dementia, senile dementia, frontotemporal dementia, Lewy body dementia, Parkinson's disease, Huntington's disease, neurogenic bladder, overactive bladder, nervous pollakiuria, urge incontinence, reflex incontinence, overflow incontinence, amyotrophic lateral sclerosis, cerebral hemorrhage, cerebral infarction, brain tumor, brain damage, spinal cord injury, Down syndrome, and hyperactivity disorder.

As the active component in the medical drug provided by the present invention, a substance selected from a group consisting of a free compound of general formula (1), a physiologically acceptable salt thereof, a hydrate thereof, and a solvate thereof may be used. Generally, the medical drug of the present invention is provided in a form of a pharmaceutical composition containing the active component and formulation additives (such as a carrier and a diluent).

The administration route of the medical drug of the present invention is not specifically limited and may be any of oral administration or parenteral administration (such as intramuscular administration, intravenous administration, subcutaneous administration, intraperitoneal administration, mucosal administration through nasal cavity and the like, and inhalation administration). The form of the medical drug of the present invention is not specifically limited. Examples of the preparation for oral administration include tablets, capsules, fine granules, powders, granules, liquids, and syrups, and examples of the preparation for parenteral administration include injections, drops, suppositories, inhalations, transmucosal absorption agents, transdermal absorption agents, nasal drops, ear drops, and eye drops.

Each of the form, the formulation additives to be used, and the formulation method for the medical drug of the present invention can be properly selected by a person skilled in the art. The dose of the medical drug of the present invention can be properly selected by comprehensively considering the sex, the age or weight, the severity of a symptom, the administration object such as prevention or treatment, the presence or absence of complicated symptoms, and the like of a patient. As for the dose, typically, in the case of the oral administration for adults, about 0.05 mg to 2 g, preferably about 0.1 mg to 1 g may be administered per day divided into 1 to 5 doses. In the case of the parenteral administration for adults, about 0.01 mg to 1 g, preferably about 0.05 mg to 0.5 mg may be administered per day divided into 1 to 5 doses.

The present invention will be further described in detail with reference to Examples and Reference Examples, but the present invention is not limited thereto.

### Examples

### Reference Example 1

### Synthesis of 5-hydroxy-1-[3-(fuan-3-yl)-3-oxo-prop-1-yl]-benzocyclobutene-1-carbonitrile ethylene ketal (Compound 18)

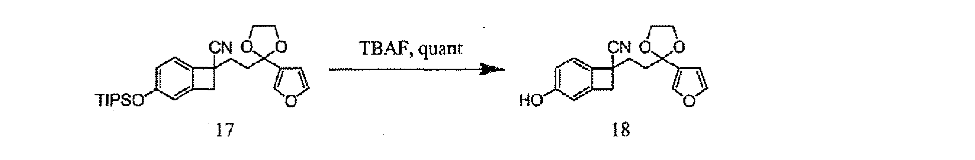

Under an argon atmosphere, to a THE solution (50 mL) of 5-triisopropylsilyloxy-1-[3-(furan-3-yl)-3-oxo-prop-1-yl]-benzocyclobutene-1-carbonitrile ethylene ketal (hereinafter called Compound 17) (542 mg, 1.16 mmol)) (Heterocycles 2004, 62, 207-211), tetra-n-butylammonium fluoride (1 M THF solution, 1.27 mL, 1.27 mmol) was added dropwise at 0°C, and the whole was stirred at room temperature for 0.5 hour. The reacted solution was diluted with a saturated aqueous ammonium chloride solution and extracted with chloroform. The organic phase was dried over magnesium sulfate and filtered, and then the solvent was removed by evaporation. The residue was purified by silica gel column chromatography (hexane:acetone = 3:2) to produce 5-hydroxy-1-[3-(furan-3-yl)-3-oxo-prop-1-yl]-benzocyclobutene-1-carbonitrile ethylene ketal (hereinafter called Compound 18) (362 mg, quant.) as yellow oil.

¹H-NMR (270 MHz, CDCl₃) δ: 1.94-2.10 (2H, m), 2.14-2.24 (2H, m), 3.14 (1H, d, J=14 Hz), 3.59 (1H, d, J=14 Hz), 3.85-4.05 (4H, m), 6.31 (1H, dd, J=1.0, 1.7 Hz), 6.60 (1H, m), 6.67 (1H, dd, J=2.3, 8.2 Hz), 6.99 (H, d, J=8.2 Hz), 7.34-7.38 (2H, m); ¹³C-NMR (67.5 MHz, CDCl₃) δ: 31.5, 36.0, 41.4, 42.1, 63.6, 64.8, 106.9, 108.5, 111.1, 115.7, 121.8, 123.0, 127.1, 134.8, 140.1, 142.1, 143.6, 157.3; IR (neat): 3389, 2234 cm⁻¹; MS (EI) m/z 311 (M⁺); HRMS (EI) Calcd for C₁₈H₁₇NO₄: 311.1158, Found: 311.1135.

### Reference Example 2

### Syntheses of 5-trifluoromethanesulfonyloxy-1-[3-(furan-3-yl)-3-oxo-pro-1-yl]-benzocyclobutene-1-carbonitrile ethylene ketal (Compound 19)

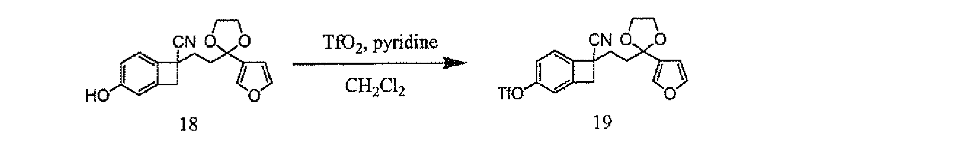

Under an argon atmosphere, to a dichloromethane solution (15 mL) of Compound 18 (362 mg, 1.16 mmol), pyridine (0.281 mL, 3.48 mmol) was added and stirred. To the mixture, a dichloromethane solution (2 mL) of trifluoromethanesulfonic anhydride (0.390 mL, 2.32 mmol) was added dropwise at 0°C, and the whole was stirred for 0.5 hour. Water was added to stop the reaction, the reacted solution was extracted with dichloromethane, the organic phase was dried over magnesium sulfate and filtered, and then the solvent was removed by evaporation. The residue was purified by silica gel column chromatography (ethyl acetate:hexane = 1:4) to produce 5-trifluoromethanesulfonyloxy-1-[3-(furan-3-yl)-3-oxo-prop-1-yl]-benzocyclobutene-1-c arbonitrile ethylene ketal (hereinafter called Compound 19) (484 mg, 94%) as colorless oil.

¹H-NMR (270 MHz, CDCl₃) δ: 2.02-2.12 (2H, m), 2.13-2.25 (2H, m), 3.28 (1H, d, J=15 Hz), 3.71 (1H, d, J=15 Hz), 3.78-4.00 (4H, m), 6.31-6.32 (1H, m), 7.09-7.10 (1H, m), 7.17 (1H, dd, J=2.3, 8.2 Hz), 7.28 (1H, d, J=8.2 Hz), 7.35-7.38 (2H, m); ¹³C-NMR (67.5 MHz, CDCl₃) δ: 31.2, 36.1, 42.1, 42.2, 106.6, 108.4, 118.0, 118.7 (q, J=321 Hz), 120.4, 122.0, 124.0, 127.1, 140.1, 143.0, 143.6, 143.6, 150.4; IR (neat): 2236 cm⁻¹; MS (EI) m/z 443 (M⁺); HRMS (EI) Calcd for C₁₉H₁₆F₃NO₆S: 443.0650, Found: 443.0621.

### Reference Example 3

### Synthesis of 8-trifluoromethanesulfonloxy-2,3,5a,10c-tetrahydro-1H,6H-5-oxa-3-oxo-acephenanthrylene-10b-carbonitrile ethylene ketal (Compound 6)

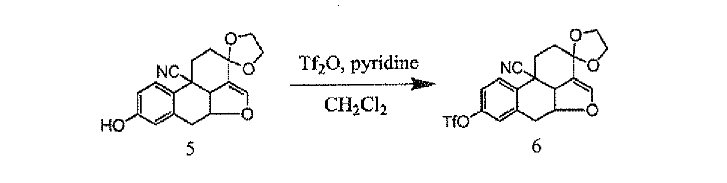

Under an argon atmosphere, to a dichloromethane solution (3 mL) of Compound 5 (56.2 mg, 0.181 mmol) (J. Org. Chem. 2004, 69, 7989-7993), pyridine (0.044 mL, 0.542 mmol) was added and stirred. To the mixture, a dichloromethane solution (1 mL) of trifluoromethanesulfonic anhydride (0.061 mL, 0.361 mmol) was added dropwise at 0°C, and the whole was stirred at 0°C for 0.5 hour. Water was added to stop the reaction, the reacted solution was extracted with dichloromethane, the organic phase was dried over magnesium sulfate and filtered, and then the solvent was removed by evaporation. The residue was purified by silica gel column chromatography (dichloromethane:hexane = 10:1) to produce Compound 6 (72.1 mg, 90%) as a white solid (mp 127 to 129°C).

¹H-NMR (270 MHz, CDCl₃) δ: 1.69-1.87 (2H, m), 2.53 (1H, td, J=4.6, 15 Hz), 2.78-2.86 (1H, m), 3.14 (1H, dd, J=2.3, 16 Hz), 3.25 (1H, dd, J=3.3, 16 Hz), 3.78-3.99 (5H, m), 5.26 (1H, ddd, J=2.6, 3.3, 11 Hz), 5.94 (1H, d, J=2.0 Hz), 7.16-7.19 (2H, m), 7.33-7.37 (1H, m); ¹³C-NMR (67.5 MHz, CDCl₃) δ: 29.6, 31.3, 33.9, 35.5, 51.1, 63.8, 65.4, 79.1, 104.3, 110.2, 118.7 (q, J=321 Hz), 120,1, 121.2, 123.2, 127.7, 131.6, 138.7, 143.2,149.3; IR (KBr): 2231 cm⁻¹; MS (EI): m/z 443 (M⁺); HRMS (EI) Calcd for C₁₉H₁₆F₃NO₆S: 443.0650, Found: 443.0664.

### Reference Example 4

### Synthesis of 8-(2,4,6-trifluorophenyl)-2,3,5a,10c-tetrahydro-1H,6H-5-oxa-3-oxo-acehenanthrylene-10b-carbonitrile ethylene petal (Compound 10)

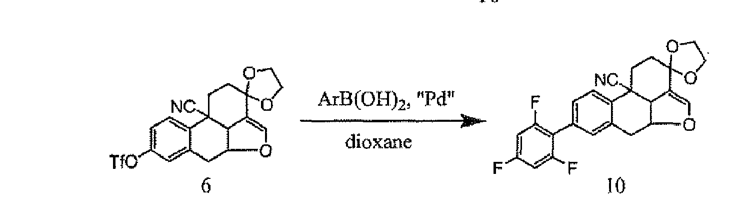

Under an argon atmosphere, into a 1,4-dioxane solution (3 mL) of Compound 6 (158 mg, 0.356 mmol), 2,4,6-trifluorophenylboric acid (68.8 mg, 0.391 mmol), potassium bromide (46.5 mg, 0.391 mmol), tripotassium phosphate hexahydrate (142 mg, 0.533 mmol), and tetrakis(triphenylphosphine)palladium (41.1 mag, 0.0356 mmol) were mixed, and the mixture was heated and refluxed for 5 hours. After being cooled, the reacted solution was diluted with dichloromethane and washed with a saturated aqueous sodium hydrogen carbonate, then the organic phase was dried over magnesium sulfate and filtered, and then the solvent was removed by evaporation. The residue was purified by silica gel column chromatography (ethyl acetate:hexane = 1:3) to produce Compound 10 (60.5 mg, 40%) as a white solid (mp 136 to 137°C).

¹H-NMR (300 MHz, CDCl₃) δ: 1.82-1.97 (2H, m), 2.56 (1H, ddd, J=5.2, 13, 15 Hz), 2.89-2.96 (H, m), 3.17 (1H, dd, J=2.5, 16 Hz), 3.30 (1H, dd, J=3.6, 16 Hz), 3.84-4.07 (5H, m), 5.33 (1H, m), 6.01 (1H, d, J=1.4 Hz), 7.26-7.40 (5H, m); ¹³C-NMR (75 MHz, CDCl₃) δ: 29.9, 31.6, 34.1, 35.9, 51.4, 63.9, 65.5, 79.9, 104.8, 110.5, 122.2, 125.7, 127.5, 128.7, 130.6, 131.0, 135.5, 143.2; IR (KBr): 2228 cm⁻¹; MY (EI) m/z 425 (M⁺); HRMS (EI) Calcd for C₂₄H₁₈F₃NO₃: 425.1239, Found: 425.1262.

### ] Reference Example 5

### Synthesis of 8-phenyl-2,3,5a,10c-tetrahdro-1H,6H-5-oxa-3-oxo-acephenanthrylene-10b carbonitrile ethylene ketal (Compound 9)

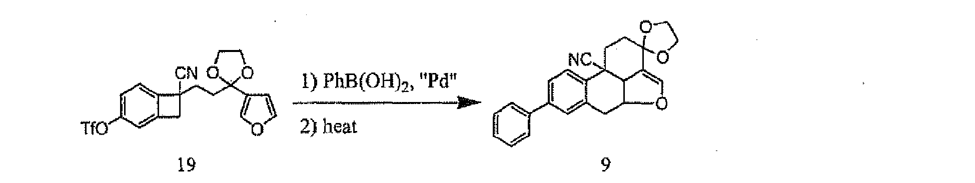

Under an argon atmosphere, into a 1,4-dioxane solution (3 mL) of Compound 19 (203 mg, 0.457 mmol), phenylboric acid (61.3 mg, 0.503 mmol), potassium bromide (59.9 mg, 0.503 mmol), tripotassium phosphate hexahydrate (183 mg, 0.686 mmol), and tetrakis(triphenylphosphine)palladium (52.9 mg, 0.0457 mmol) were mixed, and the mixture was heated and refluxed for 1 hour. After being cooled, the reacted solution was diluted with dichloromethane and washed with a saturated aqueous sodium hydrogen carbonate, then the organic phase was dried over magnesium sulfate and filtered, and then the solvent was removed by evaporation. The residue was subjected to silica gel column chromatography (ethyl acetate:hexane = 1:4) to produce a coupled compound (161 mg) as a white solid. The compound was successively dissolved in o-dichlorobenzene (25 mL), the solution was heated and refluxed for 1 hour, and then the solvent was removed by evaporation. The residue was purified by silica gel column chromatography (ethyl acetate:hexane = 1:3) to produce Compound 9 (104 mg, a two-step yield of 611%) as a white solid (mp 179°C).

¹H-NMR (270 MHz, CDCl₃) δ: 1.83-2.00 (2H, m), 2.57 (1H, ddd, J=4.9, 13, 15 Hz), 2.90-2.97 (1H, m), 3.22 (H, dd, J=2.3, 16 Hz), 3.34 (1H, dd, J=3.0, 16 Hz), 3.80-4.01 (5H, m), 5.34 (1H, ddd, J=2.3, 3.3, 11 Hz), 6.03 (1H, d, J=1.7 Hz), 7.29-7.59 (8H, m); ¹³C-NMR (67.5 MHz, CDCl₃) δ: 29.7, 31.4, 34.0, 35.5, 51.1, 63.7, 65.3, 79.7, 104.6, 10.3, 122.0, 126.1, 127.1, 127.6, 128.8, 129.1, 129.9, 135.8, 140.2, 141.5, 143.2; IR (KBr): 2226 cm⁻¹; MS (EI) m/z 371 (M⁺); HRMS (EI) Calcd for C₂₄H₂₁NO₃: 371.1522, Found: 371.1518.

### Reference Example 6

### thesis of 7-[3-(tetrahydropyran-2-yloxy)-propyl]-4,6-dimethoxy-3-oxobicyclo[4.2.0]octa-1,4-diene-7-carbonitrile (Compound 24)

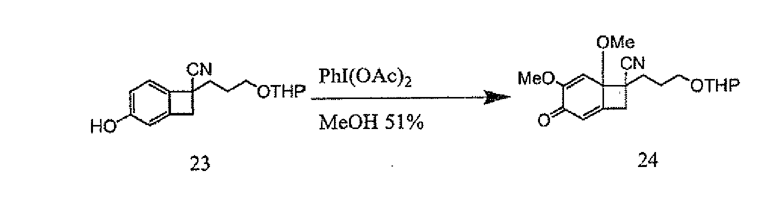

Under an argon atmosphere, to a MeOH solution (20 mL) of Compound 23 (541 mg, 1.88 mmol) (Heterocycles 2004, 62, 207-211), PhI(OAc)₂ (1.33 g, 4.14 mmol) was added, the whole was stirred at room temperature for 30 minutes, and then the reaction was stopped by a saturated aqueous NaHCO₃ solution. The organic phase extracted with CH₂Cl₂ was dried over MgSO₄ and filtered, and then the solvent was removed by evaporation. The residue was purified by silica gel column chromatography (AcOEt:hexane = 1:1) to produce Compound 24 (333 mg, 959 µmol, 51%) as yellow oil.

¹H-NMR (300 MHz, CDCl₃) δ: 1.52-2.17 (10H, m), 3.20 (1H, dd, J=1.6 Hz J=17.9 Hz), 3.37 (6H, d, J=1.37 Hz), 3.43-3.54 (2H, m), 3.60 (1H, dd, J=1.9 Hz J=17.9 Hz), 3.79-3.87 (2H, m), 4.57 (1H, s), 5.92 (1H, dd, J=1.9 Hz J=3.3 Hz), 6.33 (1H, s); ¹³C-NMR (75 MHz, CDCl₃) δ: 19.71, 19.76, 25.37, 26.03, 30.66, 34.17, 41.34, 41.92, 50.19, 50.35, 62.63, 66.13, 93.55, 99.00, 119.49, 126.80, 142.75, 155.18, 192.99; IR (neat): 1682, 2236 cm⁻¹; MS (EI): m/z 347 (M⁺); HRMS (EI): calcd for C₁₉H₂₅NO₅ 347.1733, found 347.1684.

### Reference Example 7

### Synthesis of 3-hydroxy-4-methoxy-7-[3-(tetrahydropylan-2-yloxy)-propyl]bicyclo[4.2.0]octa-1,3,5-triene-7-carbonitrile (Compound 25)

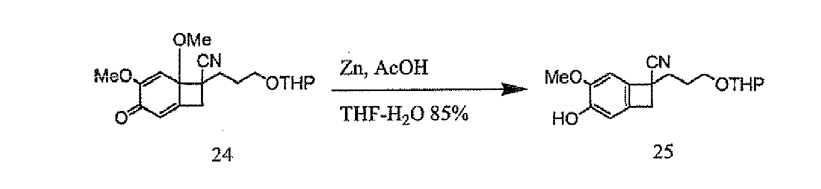

To a mixed solution of Compound 24 (464 mg, 1.34 mmol), AcOH (700 µL), THF (7.0 mL), and H₂O (7.0 mL), Zn (438 mg, 6.70 mmol) was added, the whole was stirred at room temperature for 30 minutes, and then the reaction was stopped by a saturated aqueous NaHCO₃ solution. The organic phase extracted with CH₂Cl₂ was dried over MgSO₄ and filtered, and then the solvent was removed by evaporation. The residue was purified by silica gel column chromatography (AcOEt:hexane = 1:1) to produce Compound 25 (365 mg, 1.15 mmol, 85%) as yellow oil.

¹H-NMR (300 MHz, CDCl₃) δ: 1.54-2.03 (10H, m), 3.19 (1H, d, J=13.7 Hz), 3.44-3.52 (2H, m), 3.62 (1H, d, J=13.7 Hz), 3.77-3.85 (2H, m), 3.89 (3H, s), 4.57 (1H, t, J=3.57 Hz), 5.83 (1H, s), 6.74 (1H, s), 6.76 (1H, d, J=1.9 Hz); ¹³C-NMR (75 MHz, CDCl₃) δ: 19.74, 25.48, 26.71, 30.74, 34.55, 42.05, 42.25, 56.39, 62.52, 66.65,98.91, 104.83, 110.35, 121.98, 133.31, 134.10, 147.27, 147.79; IR (neat): 2230, 341.8 cm⁻¹; MS (EI): m/z 317 (M⁺); HRMS (EI): calcd for C₁₈H₂₃NO₄ 317.1627, found 317.1604.

### Reference Example 8

### thesis of 7-[3-(tetrahydroran-2-yloxy)-propyl]-3-triisopropylsilanyloxy-4-methoxy-bicyclo[4.2.0]octa-1,3,5-triene-7-carbonitrile (Compound 26)

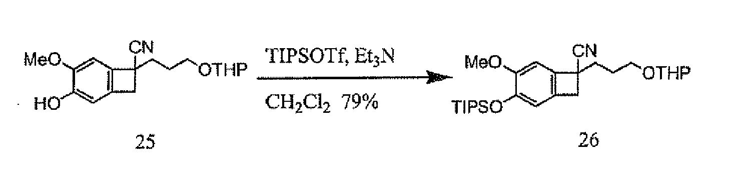

Under an argon atmosphere, to a CH₂Cl₂ solution (8.0 mL) of Compound 25 (320 mg, 1.01 mmol), Et₃N (238 µL, 1.72 mmol) and TIPS-OTf(409 µL, 1.52 mmol) were added at 0°C, and the whole was stirred at room temperature for 1 hour. Then, the reacted solution was diluted with CH₂Cl₂ and successively washed with a 3% aqueous HCl solution and a saturated aqueous NaHCO₃solution. Then the organic phase was dried over MgSO₄ and filtered, and then the solvent was removed by evaporation. The residue was purified by silica gel column chromatography (AcOEt:hexane = 1:6) to produce Compound 26(378mg, 798µmol, 79%) as colorless oil.

¹H-NMR (300 MHz, CDCl₃) δ: 1.08 (18H, d, J=7.1 Hz), 1.17-1.29 (3H, m), 1.56-2.44 (10H, m), 3.16 (1H, d, J=13.5 Hz), 3.43-3.50 (2H, m), 3.64 (1H, d, J=13.5 Hz), 3.78 (3H, s), 3.79-3.84 (2H, m), 4.57 (H, s), 6.67 (1H, s), 6.72 (1H, s); ¹³C-NMR (75 MHz, CDCl₃) δ: 12.88, 17.91, 19.63, 25.42, 26.57, 30.66, 34.46, 41.87, 42.02, 55.69, 62.36, 66.63, 98.79, 105.91, 115.91, 122.00, 132.16, 135.26, 147.56, 151.96; IR (neat): 223 cm⁻¹; MS (EI): m/z 473 (M⁺); HRMS (EI): calcd for C₂₇H₄₃NO₄Si 473.2961, found 473.2987.

### Reference Example 9

### thesis of 7-(3-hydroxypropyl)-3-triisopropylsilanyloxy-4-methoxy-bicyclo[4.2.0]-octa-1,3,5-triene-8-carbonitrile (Compound 27)

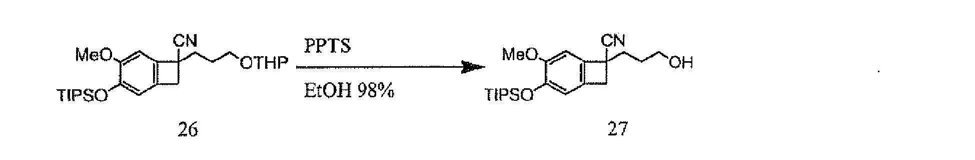

Under an argon atmosphere, to an EtOH solution (7.0 mL) of Compound 26 (329 mg, 695 µmol), PPTS (35.0 mg, 139 µmol) was added, the whole was heated and stirred at 75°C for 2 hours, and then the reaction was stopped by a saturated aqueous NaHCO₃ solution. The organic phase extracted with CH₂Cl₂ was dried over MgSO₄ and filtered, and then the solvent was removed by evaporation. The residue was purified by silica gel column chromatography (AcOEt:hexane = 1:1) to produce Compound 27 (266 mg, 683 µmol, 98%) as colorless oil.

¹H-NMR (300 MHz, CDCl₃) δ: 1.08 (18H, d, J=6.9 Hz), 1.17-1.29 (3H, m), 1.84-1.92 (2H, m), 1.97-2.06 (2H, m), 2.17 (1H, s), 3.16 (1H, d, J=13.5 Hz), 3.61 (1H, d, J=13.5 Hz), 3.73 (2H, t, J=6.0 Hz), 3.77 (3H, s), 6.67 (1H, s), 6.71 (1H, s); ¹³C-NMR (75 MHz, CDCl₃) δ: 12.86, 17.91, 29.32, 34.01, 41.79, 42.04, 55.72, 61.90, 105.86, 115.93, 122.00, 132.14, 135.15, 147.61, 151.98; IR (neat): 2232, 3446 cm⁻¹; MS (EI): m/z 389 (M⁺); HRMS (EI): calcd for C₂₂H₃₅NO₃Si 389.2386, found 389.2405.

### Reference Example 10

### Synthesis of 3-triisopropylsilanyloxy-4-methoxy-7-(3-oxopropyl)bicyclo[4.2.0]-octa-1,3,5-triene-7-carbonitrile (Compound 28)

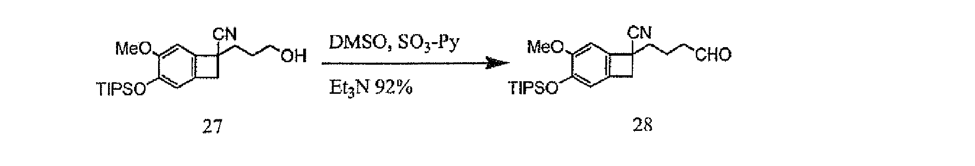

Under an argon atmosphere, to a mixed solution of Compound 27 (97.4 mg, 250 µmol), DMSO (2.0 mL), and Et₃N (2.0 mL), Py-SO₃ (199 mg, 1.25 mmol) was added at room temperature, the whole was stirred, and then the reaction was stopped by H₂O. The organic phase extracted with CH₂Cl₂ was successively washed with H₂O and a saturated aqueous NaCl solution, dried over MgSO₄, and filtered, and then the solvent was removed by evaporation. The residue was purified by silica gel column chromatography (AcOEt:hexane = 1:2) to produce Compound 28 (89.7 mg, 231 µmol, 92%) as yellow oil.

¹H-NMR (300 MHz, CDCl₃) δ: 1.08 (18H, d, J=6.9 Hz), 1.17-1.29 (3H, m), 2.23-2.32 (2H, m), 2.74-2.81 (2H, m), 3.16 (1H, d, J=13.7 Hz), 3.62 (1H, d, J=13.7 Hz), 3.78 (3H, s), 6.68 (1H, s), 6.69 (1H, s), 9.82 (1H, s); ¹³C-NMR (75 MHz, CDCl₃) δ: 12.90, 17.95, 29.63, 40.42, 41.05, 41.83, 55.77, 105.78, 116.06, 121.41, 131.98, 134.23, 147.98, 152.24, 199.58; IR (neat): 1730, 2231 cm⁻¹; MS (EI): m/z 387 (M⁺); HRMS (EI): calcd for C₂₂H₃₃NO₃Si 387.2230, found 387.2274.

### Reference Example 11

### Synthesis of 7-[3-(furan-3-yl)-3-hydroxypropyl]-3-triisopropylsilanyloxy-4-methoxy-bicyclo[4.2.0]octa-1,3,5-triene-7-carbonitrile (Compound 29)

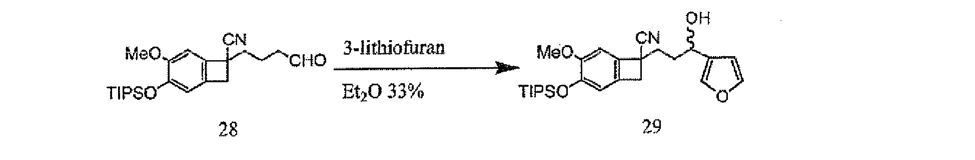

Under an argon atmosphere, to an Et₂O solution (2.0 mL) of 3-bromofuran (37.4 mL, 416 µmol), n-BuLi (1.6 M THF solution, 220 µL, 352 µmol) was added dropwise at -78°C, and the whole was stirred for 30 minutes. Then an Et₂O solution (2.0 mL) of Compound 28 (124 mg, 320 µmol) was added dropwise to the reacted solution at -78°C, the temperature was raised to 0°C, the whole was stirred for 1.5 hours, and then the reaction was stopped by a saturated aqueous NH₄Cl solution. The organic phase extracted with Et₂O was dried over MgSO₄ and filtered, and then the solvent was removed by evaporation. The residue was purified by silica gel column chromatography (AcOEt:hexane = 1:3) to produce Compound 29 (47.7 mg, 105 µmol, 33%) as yellow oil.

¹H-NMR (300 MHz, CDCl₃) δ: 1.08 (18H, d, J=6.9 Hz), 1.12-1.29 (3H, m), 1.84 (1H, s), 1.88-2.14 (4H, m), 3.14 (1H, d, J=13.5 Hz), 3.59 (1H, d, J=13.5 Hz), 3.77 (3H, s), 6.41 (1H, dt, J=1.1 Hz J=2.5 Hz), 6.69 (2H, d, J=10.7 Hz), 7.40 (2H, t, J=1.6 Hz); ¹³C-NMR (75 MHz, CDCl₃) δ: 12.92, 17.98, 33.59, 33.66, 34.19, 41.71, 41.99, 42.05, 55.82, 66.37, 105.93, 108.15, 116.03, 121.98, 128.37, 132.16, 135.07, 139.05, 143.51, 147.72, 152.09; IR (neat): 2231, 3445 cm⁻¹; MS (EI): m/z 455 (M⁺); HRMS (EI): calcd for C₂₆H₃₇NO₃Si 455.2492, found 455.2541.

### Reference Example 12

### Synthesis of 7-[3-(furan-3-yl)-3-oxopropyl]-3-triisopropylsilanyloxy-4-methoxy-bicclo[4.2.0]octa-1,3,5-triene-7-carbonitrile (Compound 30)

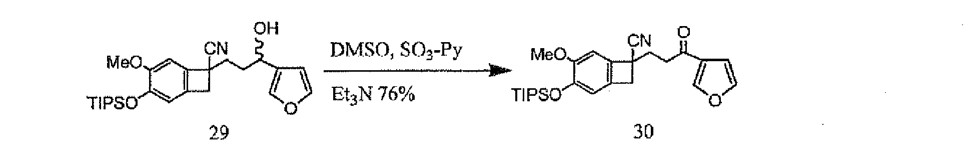

Under an argon atmosphere, to a mixed solution of Compound 29 (41.3 mg, 90.6 µmol), DMSO (1.0 mL), and Et₃N (1.0 mL), Py-SO₃ (83.6 mg, 525 µmol) was added at room temperature, the whole was stirred, and then the reaction was stopped by H₂O. The organic phase extracted with CH₂Cl₂ was successively washed with H₂O and a saturated aqueous NaCl solution, dried over MgSO₄, and filtered, and then the solvent was removed by evaporation. The residue was purified by silica gel column chromatography (AcOEt:hcxane = 1:4) to produce Compound 34 (31.4 mg, 69.2 µmol, 76%) as brown oil.

¹H-NMR (300 MHz, CDCl₃) δ: 1.48 (18H, d, J=6.9 Hz), 1.21-1.29 (3H, m), 2.37 (2H, dd, J=1.4 Hz J=7.4 Hz), 2.95-3.13 (2H, m), 3.19 (1H, d, J=13.7 Hz), 3.62 (1H, d, J=13.7 Hz), 3.77 (3H, s), 6.69 (2H, d, J=6.0 Hz), 6.77 (1H, dd, J=0.8 Hz J=1.8 Hz), 7.45 (1H, t, J=1.8 Hz), 8.07 (1H, dd, J=0.8 Hz J=1.4 Hz); ¹³C-NMR (75 MHz, CDCl₃) δ: 12.93, 17.96, 31.49, 36.72, 41.31, 41.86, 55.81, 105.86, 108.42, 116.07, 127.22, 132.08, 134.50, 144.26, 147.14, 147.93, 152.22, 192.49; IR (neat): 1683, 2230 cm⁻¹; MS (EI): m/z 454 (M⁺); HRMS (EI): calcd for C₂₆H₃₆NO₄Si 454.2414, found 454.2395.

### Reference Example 13

### Synthesis of 7-[2-(2-furan-3-yl-[1,3]dioxolane-2-yl)ethyl]-3-triisopropylsilanyloxy-4-methoxy-bicyclo[4.2.0]octa-1,3,5-triene-7-carbonitrile (Compound 31)

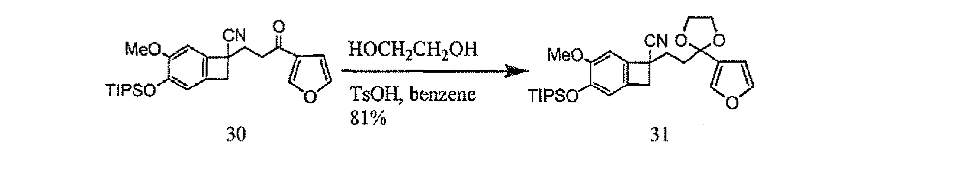

Under an argon atmosphere, to a benzene solution (5.0 mL) of Compound 30 (83.7 mg, 185 µmol), ethylene glycol (103 µL, 1.85 mmol) and TsOH (3.5 mg, 18.5 µmol) were added at room temperature, the whole was heated and refluxed for 20 hours using a Dean-Stark apparatus, and then the reaction was stopped by a saturated aqueous NaHCO₃ solution. The organic phase extracted with CH₂Cl₂ was dried over MgSO₄ and filtered, and then the solvent was removed by evaporation. The residue was purified by silica gel column chromatography (AcOEt:hexane = 1:3) to produce Compound 31 (74.8 mg, 150 µmol, 81%) as yellow oil.

¹H-NMR (300 MHz, CDCl₃) δ: 1.08 (18H, d, J=6.9 Hz), 1.12-1.31 (3H, m), 1.99-2.27 (4H, m), 3.12 (1H, d, J=13.5 Hz), 3.57 (1H, d, J=13.5 Hz), 3.77 (3H, s), 3.86-4.03 (4H, m), 6.33 (1H, q, J=0.8 Hz), 6.66 (1H, s), 6.69 (1H, s), 7.36 (1H, t, J=1.6 Hz), 7.39 (1H, t, J=0.8 Hz); ¹³C-NMR (75 MHz, CDCl₃) δ: 12.93, 17.98, 31.62, 36.00, 41.53, 41.89, 55.81, 64.80, 4.83, 05.88, 106.77, 108.42, 115.98, 127.11, 132.16, 135.09, 139.92, 143.37, 147.67, 152.06; IR (neat): 2231 cm⁻¹; MS (EI): m/z 497 (M⁺); HRMS (EI): calcd for C₂₈H₃₉NO₅Si 497.25298, found 497.2569.

### Reference Example 14

### Synthesis of 8-triisopropylsilanyloxy-9-methoxy-3-[1,3]dioxolane-2,3,5a,10c-tetrahydro-1H,6H-5-oxa-acephenanthrylene-10b-carbonitrile (Compound 32

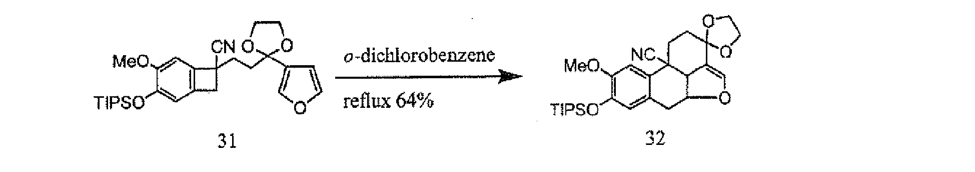

Under an argon atmosphere, an o-dichlorobenzene solution (2.0 mL) of Compound 31 (27.6 mg, 55.5 µmol) was heated and refluxed for 1.5 hours, and then the solvent was removed by evaporation. The residue was purified by silica gel column chromatography (AcOEt:hexane = 1:3) to produce Compound 32 (17.7 mg, 35.6 µmol, 64%) as colorless oil.

¹H-NMR (300 MHz, CDCl₃) δ: 1.08 (18H, d, J=6.9 Hz), 1.13-1.30 (3H, m), 1.83-1.98 (2H, m), 2.53 (1H, m), 2.84 (1H, td, J=3.6 Hz J=14.6 Hz), 3.00 (1H, dd, J=2.2 Hz J=15.9 Hz), 3.18 (1H, dd, J=3.6 Hz J=15.9 Hz), 3.79 (3H, s), 3.84-3.95 (4H, m), 3.96-4.05 (1H, m), 5.27 (1H, ddd, J=2.2 Hz J=3.6 Hz J=10.4 Hz), 6.00 (1H, d, J=2.2 Hz), 6.76 (1H, s), 6.79 (1H, s); ¹³C-NMR (75 MHz, CDCl₃) δ: 12.98, 17.99, 30.00, 31.62, 33.17, 35.53, 51.18, 55.92, 63.70, 65.32, 79.93, 104.60, 110.22, 122.24, 122.40, 122.87, 127.79, 143.01, 145.71, 149.87; IR (neat): 2228 cm⁻¹; MS (EI): m/z 497 (M⁺); HRMS (EI): calcd for C₂₈H₅₃NO₅Si 497.3662, found 497.3624.

### Reference Example 15

### Synthesis of 4-hydroxy-3-methoxy-7-[3-(tetrahydropyran-2-yloxy)-propyl]bicyclo[4.2.0]octa-1,3,5-triene-7-carbonitrile (Compound 34)

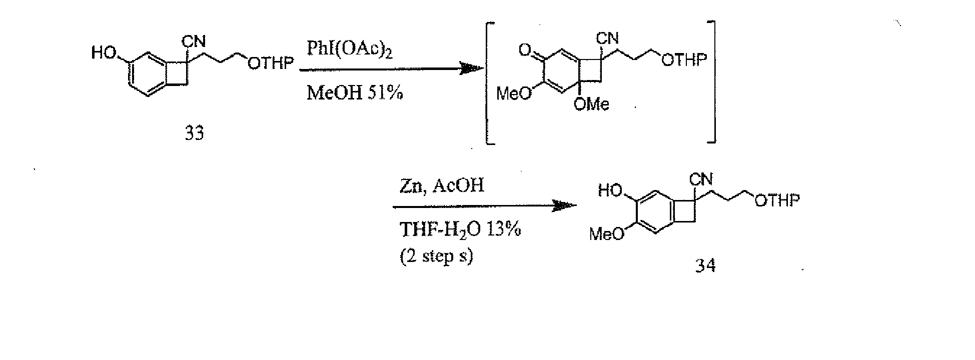

Under an argon atmosphere, to a MeOH solution (60 mL) of Compound 33 (3.03 g, 10.5 mmol) (Heterocycles 2004, 62, 207-211), PhI(OAc)₂ (7.44 g, 23.1 mmol) was added, the whole was stirred at room temperature for 30 minutes, and then the reaction was stopped by a saturated aqueous NaHCO₃ solution. The organic phase extracted with CH₂CI₂ was dried over MgSO₄ and filtered, and then the solvent was removed by evaporation. The residue was separated by silica gel column chromatography (AcOEt:hexane = 1: 1), and the solvent was removed by evaporation. To a mixed solution of the residue, AcOH (6.0 mL), THF (60.0 mL), and H₂O (60.0 mL), Zn (1.22 mg, 18.7 mmol) was added, the whole was stirred at room temperature for 30 minutes, and then the reaction was stopped by a saturated aqueous NaHCO₃ solution. The organic phase extracted with CH₂Cl₂ was dried over MgSO₄ and filtered, and then the solvent was removed by evaporation. The residue was purified by silica gel column chromatography (AcOEt:hexane = 1:2) to produce Compound 34 (447 mg, 1.41 mmol, 13%) as yellow oil.

¹H-NMR (300 MHz, CDCl₃) δ: 1.48-2.09 (10H, m), 3.18 (1H, d, J=13.5 Hz), 3.41-3.51 (2H, m), 3.61 (1H, d, J=13.5 Hz), 3.74-3.92 (2H, m), 3.87 (3H, s), 4.57 (1H, br), 5.83 (1H, s), 6.68 (1H, s), 6.80 (1H, d, J=1.4 Hz); ¹³C-NMR (75 MHz, CDCl₃) δ: 19.63, 25.45, 26.59, 30.68, 34.45, 41.99, 42.25, 56.29, 62.36, 66.63, 98.81, 106.75, 108.36, 121.80, 131.70, 135.36, 146.51, 148.48; IR (neat): 3449 cm⁻¹; MS (EI): m/z 317 (M⁺).

### Reference Example 16

### Synthesis of 7-[3-(tetrahydropyran-2-yloxy)-propyl]-4-triisopropylsilanyloxy-3-methoxy-bicyclo[4.2.0]octa-1,3,5-triene-7-carbonitrile (Compound 35)

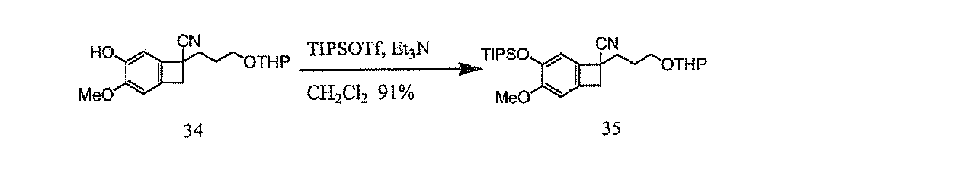

Under an argon atmosphere, to a CH₂CI₂ solution (12.0 mL) of Compound 34 (493 mg, 1.55 mmol), Et₃N (366 µL, 2.64 mmol) and TIPS-OTf (626 µL, 2.33 mmol) were added at 0°C, and the whole was stirred at room temperature for 45 minutes. Then, the reacted solution was diluted with CH₂Cl₂ and subsequently washed with a 3% aqueous HC1 solution and a saturated aqueous NaHCO₃ solution, the organic phase was dried over MgSO₄ and filtered, and then the solvent was removed by evaporation. The residue was purified by silica gel column chromatography (AcOEt:hexane = 1:4) to produce Compound 35 (666 mg, 1.41 mmol, 91%) as yellow oil.

¹H-NMR (300 MHz, CDCl₃) δ: 1.08 (18H, d, J=6.9 Hz), 1.15-1.29 (3H, m), 1.53-2.04 (10H, m), 3.18 (1H, d, J=13.7 Hz), 3.41-3.52 (2H, m), 3.61 (1H, d, J=13.7 Hz), 3.79-3.85 (2H, m), 3.77 (3H, s), 4.57 (1H, br), 6.65 (1H, s), 6.74 (1H, d, J=1.6 Hz); ¹³C-NMR (75 MHz, CDCl₃) δ: 12.96, 17.99, 19.69, 25.50, 26.62, 30.73, 34.56, 41.83, 42.21, 55.69, 62.39, 66.65, 98.79, 107.92, 113.95, 122.03, 133.11, 134.60, 146.38, 153.22; IR (neat): 2231 cm⁻¹; MS (EI): m/z 473 (M⁺).

### Reference Example 17

### Synthesis of 7-(3-hydroxypropyl)-4-triisopropylsilanyloxy-3-methoxy-bicyclo[4.2.0]octa-1,3,5-triene-7-carbonitrile (Compound 36)

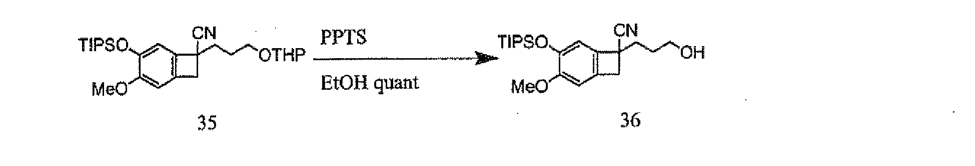

Under an argon atmosphere, to an EtOH solution (10.0 mL) of Compound 35 (666 mg, 1.41 mmol), PPTS (70.9 mg, 282 µmol) was added, the whole was heated and stirred at 75°C for 2 hours, and then the reaction was stopped by a saturated aqueous NaHCO₃ solution. The organic phase extracted with CH₂Cl₂ was dried over MgSO₄ and filtered, and then the solvent was removed by evaporation. The residue was purified by silica gel column chromatography (AcOEt:hexane = 1:2) to produce Compound 36 (576 mg, 1.48 µmol, 105%) as colorless oil.

¹H-NMR (300 MHz, CDCl₃) δ: 1.08 (18H, d, J=6.6 Hz), 1.12-1.29 (3H, m), 1.45 (1H, br), 1.80-1.90 (2H, m), 1.96-2.04 (2H, m), 3.17 (1H, d, J=13.5 Hz), 3.62 (1H, d, J=13.5 Hz), 3.72 (2H, t, J=6.0 Hz), 3.77 (3H, s), 6.65 (1H, s), 6.73 (1H, s); ¹³C-NMR (75 MHz, CDCl₃) δ: 12.96, 17.99, 29.42, 34.08, 41.75, 42.26, 55.71, 62.13, 107.95, 113.86, 121.98, 133.06, 134.50, 146.43, 153.27; IR (neat): 2232, 3437 cm⁻¹; MS (EI): m/z 389 (M⁺).

### Reference Example 18

### Synthesis of 4-triisopropylsilanyloxy-3-methoxy-7-(3-oxopropyl)bicyclo[4.2.0]octa-1,3,5-triene-7-carbonitrile (Compound 37)

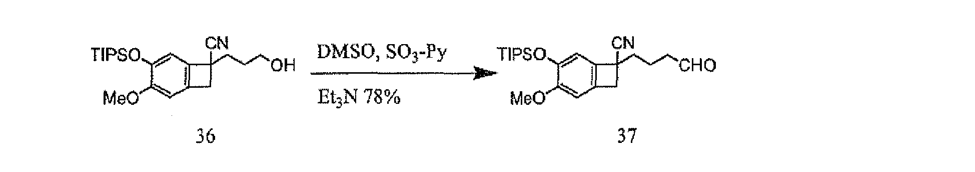

Under an argon atmosphere, to a mixed solution of Compound 36 (123 mg, 316 µmol), DMSO (2.0 mL), and Et₃N (2.0 mL), Py-SO₃ (251 mg, 1.58 mmol) was added at room temperature, the whole was stirred, and then the reaction was stopped by H₂O. The organic phase extracted with CH₂Cl₂ was successively washed with H₂O and a saturated aqueous NaCl solution, dried over MgSO₄, and filtered, and then the solvent was removed by evaporation. The residue was purified by silica gel column chromatography (AcOEt:hexane = 1:3) to produce Compound 37 (94.9 mg, 245 µmol, 78%) as yellow oil.

¹H-NMR (300 MHz, CDCl₃) δ: 1.07 (18H, d, J=6.9 Hz), 1.17-1.31 (3H, m), 2.16-2.35 (2H, m), 2.75 (2H, t, J=7.7 Hz), 3.17 (1H, d, J=13.5 Hz), 3.63 (1H, d, J=13.5 Hz), 3.76 (3H, s), 6.66 (1H, s), 6.71 (1H, s), 9.81 (1H, s); ¹³C-NMR (75 MHz, CDCl₃) δ: 12.90, 17.93, 29.56, 40.39, 40.95, 41.99, 55.61, 107.90, 113.71, 121.33, 132.82, 133.47, 146.54, 153.50, 199.48; IR (neat): 1726, 2231 cm⁻¹; MS (EI): m/z 387 (M⁺).

### Reference Example 19

### Synthesis of 7-[3-(furan-3-yl)-3-hydroxypropyl]-4-triisopropylsilanyloxy-3-methoxy-bicyclo[4.2.0]octa-1,3,5-triene-7-carbonitrile (Compound 38)

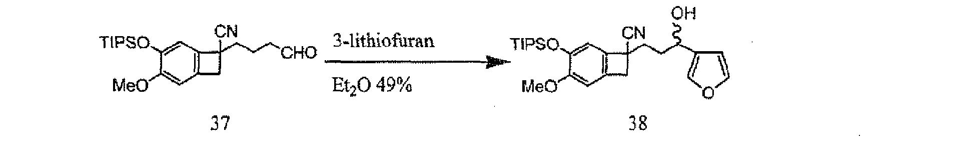

Under an argon atmosphere, to an Et₂O solution (2.0 mL) of 3-bromofuran (49.4 mL, 549 µmol), n-BuLi (1.6 M THF solution, 298 µL, 476 µmol) was added dropwise at -78°C, and the whole was stirred for 30 minutes. Then, an Et₂O solution (2.0 mL) of Compound 37 (142 mg, 366 µmol) was added dropwise to the reacted solution at -78°C, the temperature was raised to 0°C, the whole was stirred for 1.5 hours, and then the reaction was stopped by a saturated aqueous NH₄Cl solution. The organic phase extracted with Et₂O was dried over MgSO₄ and filtered, and then the solvent was removed by evaporation. The residue was purified by silica gel column chromatography (AcOEt:hexane = 1:3) to produce Compound 38 (81.6 mg, 179 µmol, 49%) as colorless oil.

¹H-NMR (300 MHz, CDCl₃) δ: 1.08 (18H, d, J=6.9 Hz), 1.13-1.29 (3H, m), 1.84-2.17 (5H, m), 3.15 (1H, dd, J=3.3 Hz J=13.7 Hz), 3.61 (1H, d, J=13.7 Hz), 3.77 (3H, s), 4.70-4.74 (1H, m), 6.38-6.40 (1H, m), 6.65 (1H, s), 6.73 (1H, s), 7.39 (2H, t, J=1.6 Hz); ¹³C-NMR (75 MHz, CDCl₃) δ_{:} 12.96, 17.99, 33.67, 34.27, 41.62, 42.17, 55.71, 66.44, 107.97, 108.11, 113.83, 121.91, 128.37, 133.05, 134.41, 139.00, 143.51, 146.43, 153.30; IR (neat): 2232, 3478 cm⁻¹; MS (EI): m/z 455 (M⁺).

### Reference Example 20

### Synthesis of 7-[3-(furan-3-yl)-3-oxopropyl]-4-triisopropylsilanyloxy-3-methoxy-bicyclo[4.2.0]octa-1,3,5-triene-7-carbonitrile (Compound 39)

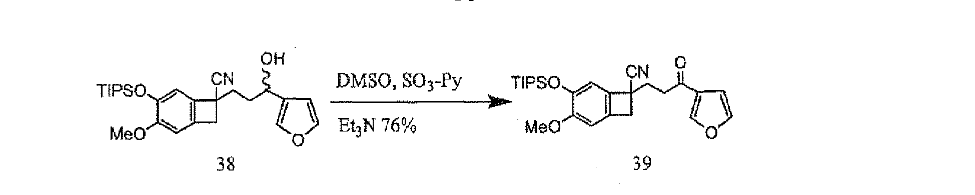

Under an argon atmosphere, to a mixed solution of Compound 38 (167 mg, 367 µmol), DMSO (2.0 mL), and Et₃N (2.0 mL), Py-SO₃ (293 mg, 1.84 mmol) was added at room temperature, the whole was stirred, and then the reaction was stopped by H₂O. The organic phase extracted with CH₂Cl₂ was successively washed with H₂O and a saturated aqueous NaCl solution, dried over MgSO₄, and filtered, and then the solvent was removed by evaporation. The residue was purified by silica gel column chromatography (AcOEt:hexane = 1:4) to produce Compound 39 (126 mg, 278 µmol, 76%) as yellow oil.

¹H-NMR (300 MHz, CDCl₃) δ: 1.07 (18H, d, J=7.1 Hz), 1.12-1.28 (3H, m), 2.32-2.38 (2H, m), 2.99-3.05 (2H, m), 3.20 (1H, d, J=13.7 Hz), 3.63 (1H, d, J=13.7 Hz), 3.76 (3H, s), 6.66 (1H, s), 6.72 (1H, s), 6.76 (1H, dd, J=0.8 Hz J=1.9 Hz), 7.44 (1H, dd, J=1.4 Hz J=1.9 Hz), 8.06 (1H, dd, J=0.8 Hz J=1.4 Hz); ¹³C-NMR (75 MHz, CDCl₃) δ: 12.90, 17.93, 31.46, 36.71, 41.21, 42.04, 55.63, 107.92, 108.39, 113.71, 121.59, 127.19, 132.92, 133.71, 144.21, 146.49, 147.07, 153.43, 192.41; IR (neat): 1683, 2231 cm⁻¹; MS (El): m/z 453 (m⁺).

### Reference Example 21

### Synthesis of 7-[2-(2-furan-3-yl-[1,3]dioxolane-2-yl)ethyl]-4-triisopropylsilanyloxy-3-methoxy-bicyclo[4.2.1]octa-1,3,5-triene-7-carbonitrile (Compound 40)

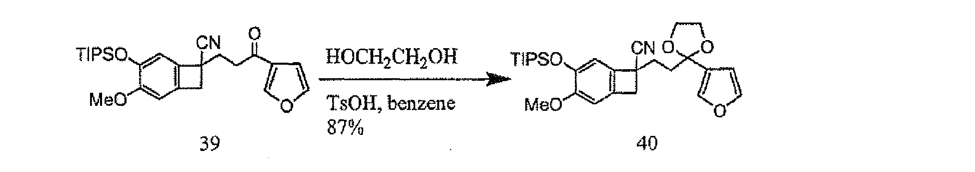

Under an argon atmosphere, to a benzene solution (7.0 mL) of Compound 39 (119 mg, 262 µmol), ethylene glycol (147 µL, 2.62 mmol) and TsOH (5.0 mg, 26.2 µmol) were added at room temperature, the whole was heated and refluxed for 13 hours using a Dean-Stark apparatus, and then the reaction was stopped by a saturated aqueous NaHCO₃ solution. The organic phase extracted with CH₂Cl₂ was dried over MgSO₄ and filtered, and then the solvent was removed by evaporation. The residue was purified by silica gel column chromatography (AcOEt:hexane = 1:3) to produce Compound 40 (114 mg, 229 µmol, 87%) as yellow oil.

¹H-NMR (300 MHz, CDCl₃) δ: 1.08 (18H, d, J=6.6 Hz), 1.19-1.29 (3H, m), 1.97-2.19 (4H, m), 3.13 (1H, d, J=13.5 Hz), 3.58 (1H, d, J=13.5 Hz), 3.76 (3H, s), 3.86-4.01 (4H, m), 6.31 (1H, dd, J=1.1 Hz J=1.6 Hz), 6.63 (1H, s), 6.71 (1H, s), 7.36 (1H, t, J=1.6 Hz), 7.37 (1H, dd, J=1.1 Hz J=1.6 Hz); ¹³C-NMR (75 MHz, CDCl₃) δ: 12.93, 17.96, 31.60, 35.97, 41.37,42.02, 55.66, 64.78, 106.74, 107.92, 108.37, 113.84, 121.80, 127.11, 133.01, 134.32, 139.87, 143.32, 146.35, 153.24; IR (neat): 2231 cm⁻¹; MS (EI): m/z 497 (M⁺).

### Reference Example 22

### Synthesis of 9-triisopropylsilanyloxy-8-methoxy-3-[1,3]dioxolane-2,3,5a,10c-tetrahydro-1H,6H-5-oxa-acephenanthrylene-10b-carbonitrile (Compound 41)

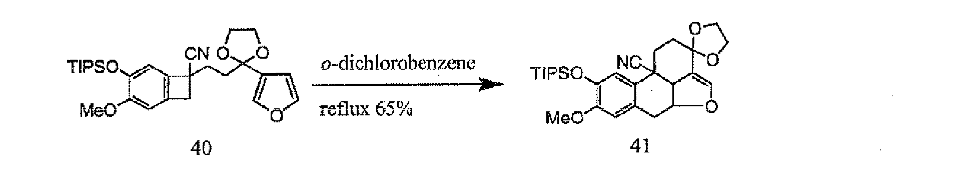

Under an argon atmosphere, an o-dichloro-benzene solution (2.0 mL) of Compound 40 (68.8 mg, 138 µmol) was heated and refluxed for 1 hour, and then the solvent was removed by evaporation. The residue was purified by silica gel column chromatography (AcOEt:hexane = 1:3) to produce Compound 41 (44.6 mg, 89.6 µmol, 65%) as a white solid (mp 131 to 133°C).

¹H-NMR (300 MHz, CDCl₃) δ: 1.08 (18H, d, J=6.6 Hz), 1.12-1.30 (3H, m), 1.79-1.95 (2H, m), 2.45-2.56 (1H, m), 2.75 (1H, dt, J=3.6 Hz J=14.6 Hz), 3.05 (1H, dd, J=2.2 Hz J=15.9 Hz), 3.22 (1H, dd, J=3.6 Hz J=15.9 Hz), 3.81 (3H, s), 3.84-3.94 (4H, m), 3.95-4.05 (1H, m), 5.28 (1H, ddd, J=2.2 Hz J=3.6 Hz J=10.7 Hz), 6.01 (1H, d, J=2.2 Hz), 6.75 (1H, s), 6.82 (1H, s); ¹³C-NMR (75 MHz, CDCl₃) δ: 12.96, 17.96, 29.92, 31.34, 33.53, 35.27, 51.03, 55.42, 63.68, 65.32, 79.93, 104.59, 110.43, 113.79, 117.85, 122.19, 122.22, 128.13, 142.82, 144.42, 150.67; IR (neat): 2358 cm⁻¹; MS (EI): m/z 497 (M⁺).

### Example 1

### Synthesis of 9-hydroxy-2,3,5a, 10c-tetrahydro-1H,6H-5-oxa-3-oxo-acephenanthrylene-10b-carbonitrile ethylene ketal (Compound 11)

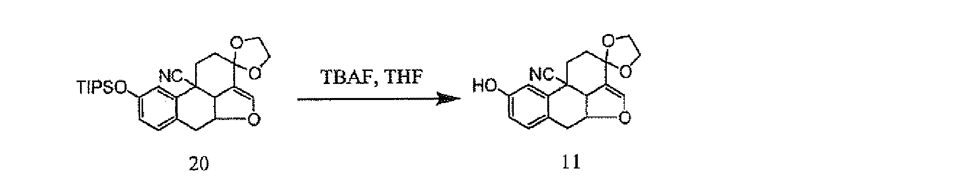

Under an argon atmosphere, to a THF solution (2.0 mL) of 8-triisopropylsilyloxy-2,3,5a, 10c-tetrahydro-1H,6H-5-oxa-3-oxo-acephenanthrylene-10b-carbonitrile ethylene ketal (hereinafter called Compound 20) (19 mg, 0.041 mmol) (Bioorg. Med. Chem. 2007, 15, 424-432), tetra-n-butylammonium fluoride (1 M THF solution, 0.053 mL, 0.053 mmol) was added dropwise at 0°C, and then the whole was stirred at room temperature for 1.5 hours. The reacted solution was diluted with a saturated aqueous ammonium chloride solution and extracted with dichloromethane, the organic phase was dried over magnesium sulfate and filtered, and then the solvent was removed by evaporation. The residue was purified by silica gel column chromatography (hexane:ethyl acetate = 1:1) to produce Compound 11 (12 mg, 95%) as a white solid (mp 61 to 63°C).

¹H-NMR (300 MHz, CDCl₃) δ: 1.82-1.98 (2H, m), 2.47-2.58 (1H, m), 2.80 (1H, td, J=3.3, 15 Hz), 3.11 (1H, dd, J=2.2, 16 Hz), 3.18 (1H, dd, J=3.3, 16 Hz), 3.87-3.96 (4H, m), 3.98-4.04 (1H, m), 5.29 (1H, td, J=2.8, 11 Hz), 6.03 (1H, d, J=1.9 Hz), 6.74 (1H, dd, J=2.5, 8.2 Hz), 6.86 (1H, d, J=2.2 Hz), 7.12 (1H, d, J=8.2 Hz); ¹³C-NMR (75 MHz, CBCl3) δ: 29.7, 31.5, 32.9, 35.8, 51.0, 63.7, 65.3, 79.8, 104.6, 110.2, 113.1, 115.5, 121.8, 127.0, 131.3, 132.0, 143.0, 155.0; IR (KBr): 3396, 2232 cm⁻¹; MS (EI): m/z 311 (M⁺); HRMS (El): calcd for C₁₈H₁₇NO₄: 311.1158, Found: 311.1152.

### Example 2

### Synthesis of 8-hydroxy-9-methoxy-3-[1,3]dioxolane-2,3,5a,10c-tetrahydro-1H,6H-5-oxa-3-oxo-acephenanthrylene-10b-carbonitrile ethylene ketal (Compound 21)

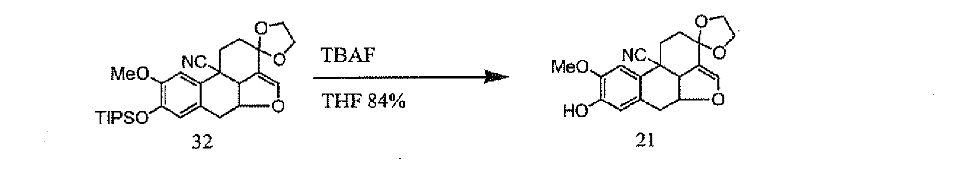

Under an argon atmosphere, to a THF solution (2.0 mL) of Compound 32 (17.7 mg, 35.6 µmol), TBAF (1.0 M THF solution, 46.3 µL, 46.3 µmol) was added dropwise at 0°C, the whole was stirred at room temperature for 40 minutes, and then the reaction was stopped by a saturated aqueous NH₄Cl solution. The organic phase extracted with CH₂Cl₂ was washed with H₂O dried over MgSO₄, and filtered, and then the solvent was removed by evaporation. The residue was purified by silica gel column chromatography (AcOEt:hexane = 1:1) to produce Compound 21 (10.2 mg, 29.9 µmol, 84%) as colorless oil.

¹H-NMR (300 MHz, CDCl₃) δ: 1.84-1.97 (2H, m), 2.55 (1H, ddd, J=5.5 Hz J=12.4 Hz J=14.8 Hz), 2.84 (1H, dt, J=3.3 Hz J=14.8 Hz), 3.05 (1H, dd, J=2.2 Hz J=15.9 Hz), 3.19 (1H, dd, J=3.6 Hz J=15.9 Hz), 3.88 (3H, s), 3.82-3.95 (4H, m), 3.97-4.06 (1H, m), 5.28 (1H, ddd, J=2.2 Hz J=3.6 Hz J=10.4 Hz), 5.66 (1H, s), 6.04 (1H, d, J=2.2 Hz), 6.77 (1H, s), 6.85 (1H, s); ¹³C-NMR (75 MHz, CDCl₃) δ: 30.02, 31.60, 35.53, 51,08, 56.21, 63.73, 65.35, 79.83, 104.55, 108.37, 110.10, 116.56, 121.85, 128.61, 143.22, 145.65, 145.71; IR (neat): 3536 cm⁻¹; MS (EI): m/z 341 (M⁺); HRMS (EI): calcd for C₁₉H₁₉NO₅ 341.1297, found 341.1263_{.}

### Example 3

### Synthesis of 9-hydroxy-8-methoxy-3-[1,3]dioxolane-2,3,5a, 10c-tetrahydro-1H,6H-5-oxa-3-oxo-acephenanthrylene-10b-carbonitrile ethylene ketal (Compound 22)

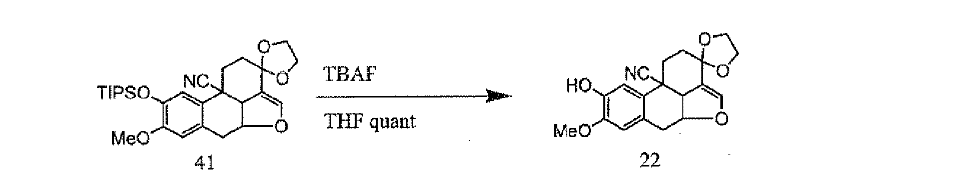

Under an argon atmosphere, to a THF solution (2.0 mL) of Compound 41 (41.4 mg, 83.2 µmol), TBAF (1.0 M THF solution, 108 µL, 108 µmol) was added dropwise at 0°C, the whole was stirred at room temperature for 30 minutes, and then the reaction was stopped by a saturated aqueous NH₄Cl solution. The organic phase extracted with CH₂Cl₂ was washed with H₂O dried over MgSO₄, and filtered, and then the solvent was removed by evaporation. The residue was purified by silica gel column chromatography (AcOEt:hexane = 1:1) to produce Compound 22 (30.6 mg, 89.6 µmol, 100%) as colorless oil.

¹H-NMR (300 MHz, CDCl₃) δ: 1.80-1.98 (2H, m), 2.50 (1H, dt, J=4.4 Hz J=14.6 Hz), 2.80 (1H, td, J=3.3 Hz J=14.6 Hz), 3.06 (1H, dd, J=2.2 Hz J=15.9 Hz), 3.22 (1H, dd, J=3.3 Hz J=15.9 Hz), 3.88 (3H, s), 3.82-3.94 (4H, m), 3.95-4.13 (1H, m), 5.28 (1H, ddd, J=2.2 Hz J=3.3 Hz J=10.4 Hz), 5.62 (1H, s), 6.04 (1H, d, J=2.2 Hz), 6.76 (1H, s), 6.90 (1H, s); ¹³C-NMR (75 MHz, CDCl₃) δ: 29.74, 31.31, 33.42, 35.26, 50.87, 55.86, 63.59, 65.19, 79.74, 104.48, 110.32, 112.11, 112.40, 121.93, 123.03, 126.85, 142.80, 144.58, 146.25; IR (neat): 3444 cm⁻¹; MS (EI): m/z 341 (M⁺).

### Example 4

### Measurement of Neuronal Cell Death Induced by Aβ(25-35)

Cerebral cortical neurons of SD rats (embryonic day 18) were primary cultured. The neurons were seeded in a 24-well culture dish at a density of 2.86 × 10⁵ cells/cm², and after 3 days, simultaneously treated with 10 µM partial peptide including the 25 to 35-amino acid residue of Aβ (hereinafter called Aβ(25-35)) and each of Compounds 1 to 5 (100 nM). After another 4 days, 6 µM calcein AM was added, the whole was incubated for 40 minutes at 37°C, and the fluorescence intensity at 485 nm was measured so that the uptake of calcein AM into the neurons was calculated to determine the cell viability. The group without the Aβ(25-35) treatment and the administration of any medical agents was defined as a control group, and the group with the Aβ(25-35) treatment but without the administration of any medical agents was defined as a vehicle group.

Separately, the neurons were seeded in an 8-well culture slide at a density of 2.14 × 10⁵ cells/cm², and after 5 days, simultaneously treated with 10 µM Aβ(25-35) and Compound 11 (100 nM). After another 4 days, 6 µM calcein AM was added, and the whole was incubated for 40 minutes at 37°C. Then the neurons were fixed, and the fluorescence image was obtained under a fluorescence microscope AX-80. The uptake of calcein AM into the neurons was quantitatively analyzed with an ATTO Densitograph (ATTO, Tokyo, Japan) to determine the cell viability.
Here, the data is shown by mean value ± standard error. The significance test was carried out by one way ANOVA (analysis of variance) and then by Dunnett's test as post hoc test. The significance level was 5%.

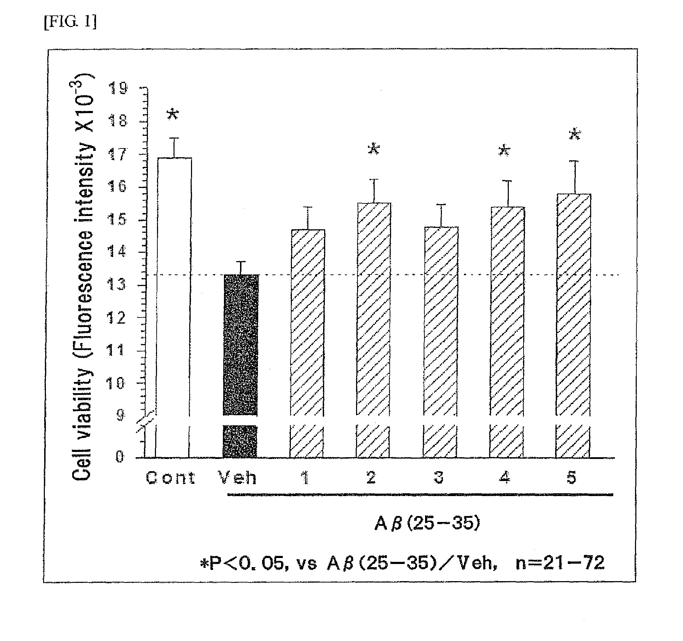

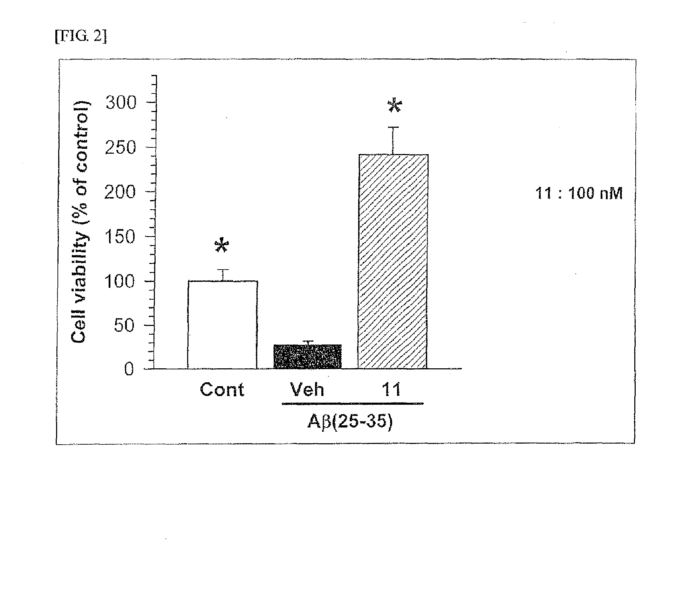

The results are shown in FIG. 1 and FIG. 2. The vehicle group was ascertained to have significantly low cell viability. In contrast, each of the groups treated with Compounds was ascertained to have higher cell viability comparing with that of the vehicle group. In particular, the group treated with Compound 11 was ascertained to have a cell viability remarkably higher than that of the control group.

### Example 5

### Measurement of Dendrite Atrophy Induced by Aβ(25-35)

Cerebral cortical neurons of SD rats (embryonic day 20) were primary cultured. The neurons were seeded in an 8-well culture slide at a density of 1.43 × 10⁵ cells/cm², and after 3 days, treated with 10 µM Aβ(25-35) and each of Compounds 5 and 11 (10 µM). After another 5 days, immunostaining was carried out. The used primary antibody was a rabbit polyclonal antibody against MAP2 that is a marker protein of the dendrite (a dilution degree of 1000; Chemicon, Temecula, CA, USA). The used secondary antibody was Alexa Fluor 568 labeled goat anti-rabbit IgG (a dilution degree of 300; Molecular Probes, Carlsbad, CA, USA). The neurons were observed under a fluorescence microscope AX-80 (Olympus, Tokyo, Japan) and the fluorescence image was obtained. The average length of the dendrites per neuron was measured with Neurocyte Ver. 1.5 (Toyobo, Osaka, Japan). The group without the Aβ(25-35) treatment and the administration of any medical agents was defined as a control group, and the group with the Aβ(25-35) treatment but without the administration of any medical agents was defined as a vehicle group.

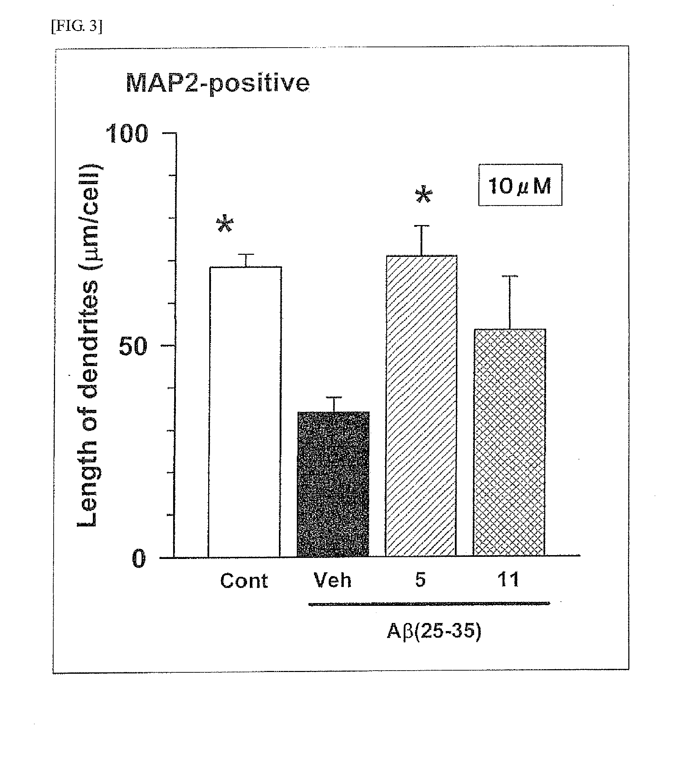

The results are shown in FIG. 3. The vehicle group was ascertained to have the dendrite atrophy. In contrast, each of the groups treated with Compounds was ascertained to have dendrite outgrowth effect as remarkably high as that of the control group.

### Example 6

### Measurement of Axon Atrophy Induced by Aβ(25-35)

Cerebral cortical neurons of SD rats (embryonic day 20) were primary cultured. The neurons were seeded in an 8-well culture slide at a density of 2.14 × 10⁵ cells/cm², and after 3 days, treated with 10 µM Aβ(25-35) and each of Compounds 8 (1 µM) and 11 (1 and 10 µg/ml). After another 4 days, immunostaining was carried out. The used primary antibody was a rabbit polyclonal antibody against phosphorylated neurofilament H that is a marker protein of the axon (a dilution degree of 300; Sternberger Monoclonals, MD, USA). The used secondary antibody was Alexa Fluor 488 labeled goat anti-rabbit IgG (a dilution degree of 300; Molecular Probes, Carlsbad, CA, USA). The neurons were observed under a fluorescence microscope AX-80 (Olympus, Tokyo, Japan) and the fluorescence image was obtained. The average length of the axons per neuron was measured with Neurocyte Ver. 1.5 (Toyobo, Osaka, Japan). The group without the Aβ(25-35) treatment and the administration of any medical agents was defined as a control group, and the group with the Aβ(25-35) treatment but without the administration of any medical agents was defined as a vehicle group.

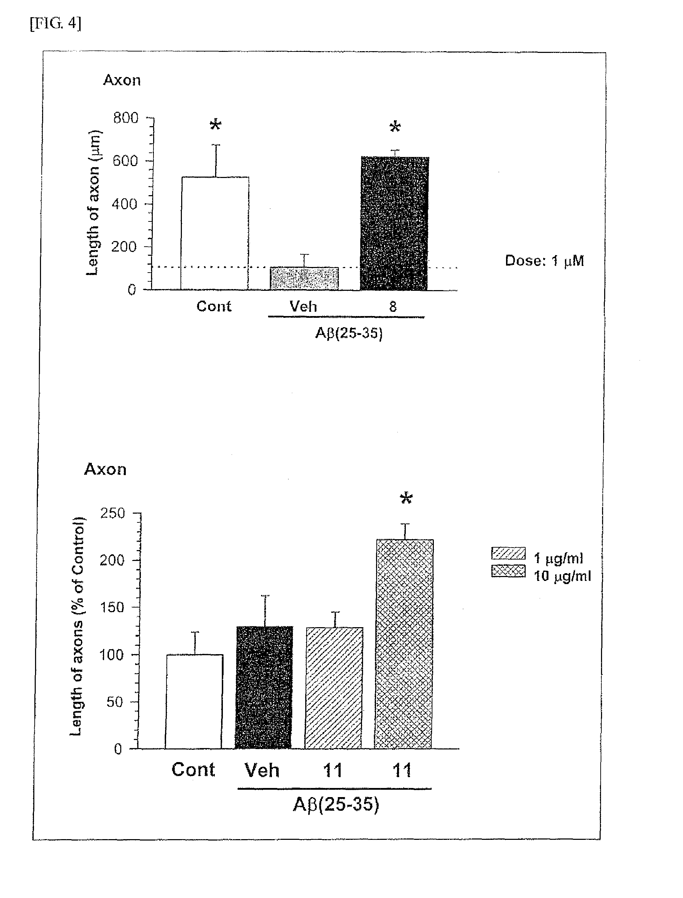

The results are shown in FIG. 4. The vehicle group was ascertained to have the axon atrophy. In contrast, each of the groups treated with Compounds was ascertained to have axon outgrowth effect as remarkably high as that of the control group.

### Example 7

### Measurement of Dendrite Atrophy Induced by Aβ(1-42)

Cerebral cortical neurons of SD rats (embryonic day 18) were primary cultured. The neurons were seeded in an 8-well culture slide at a density of 2.6 × 10⁵ cells/cm^{2.} After 5 days, the neurons were treated with 5 µM Aβ(1-42), and after another 3 days, treated with each of Compounds 5, 11, 21, and 22 (each 1 µM). After another 5 days, immunostaining was carried out. The used primary antibody was a rabbit polyclonal antibody against MAP2 that is as a marker protein of the dendrite (a dilution degree of 500; Chemicon, Temecula, CA, USA). The used secondary antibody was Alexa Fluor 568 labeled goat anti-rabbit IgG (a dilution degree of 300; Molecular Probes, Carlsbad, CA, USA). The neurons were observed under a fluorescence microscope AX-80 (Olympus, Tokyo, Japan) and the fluorescence image was obtained. The average length of the dendrites per neuron was measured with Neurocyte Ver. 1.5 (Toyobo, Osaka, Japan). The group without the Aβ(1-42) treatment and the administration of any medical agents was defined as a control group, and the group with the Aβ(1-42) treatment but without the administration of any medical agents was defined as a vehicle group.

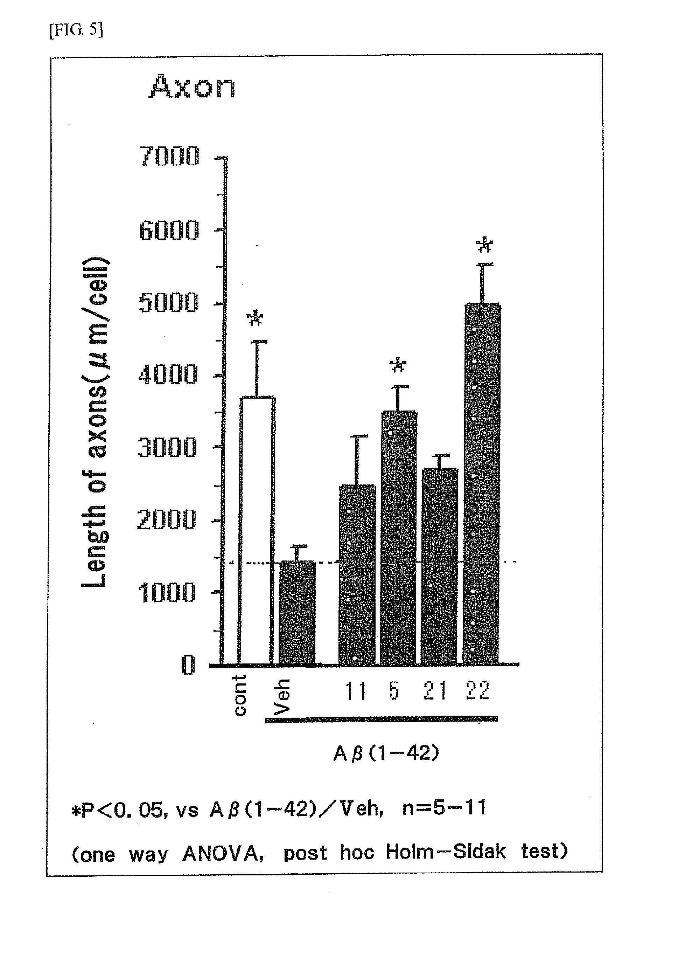

The results are shown in FIG. 5. The vehicle group was ascertained to have the dendrite atrophy. In contrast, each of the groups treated with Compounds was ascertained to have dendrite outgrowth effect as remarkably high as that of the control group. In particular, the group treated with Compound 22 was ascertained to have a dendrite outgrowth effect remarkably higher than that of the control group.

### Example 8

### Measurement of Axon Atrophy Induced by Aβ(1-42)

Cerebral cortical neurons of SD rats (embryonic day 18) were primary cultured. The neurons were seeded in an 8-well culture slide at a density of 2.6 × 10⁵ cells/cm², after 5 days, treated with 5 µM Aβ(1-42), and after another 3 days, treated with each of Compounds 5, 11, 21, and 22 (each 1 µM). After another 5 days, immunostaining was carried out. The used primary antibody was a mouse monoclonal antibody against phosphorylated neurofilament H that is a marker protein of the axon (a dilution degree of 500; Sternberger Monoclonals, MD, USA). The used secondary antibody was Alexa Fluor 488 labeled goat anti-rabbit IgG (a dilution degree of 300; Molecular Probes, Carlsbad, CA, USA). The neurons were observed under a fluorescence microscope AX-80 (Olympus, Tokyo, Japan) and the fluorescence image was obtained. The average length of the axons per neuron was measured with Neurocyte Ver. 1.5 (Toyobo, Osaka, Japan). The group without the Aβ(1-42) treatment and the administration of any medical agents was defined as a control group, and the group with the Aβ(1-42) treatment but without the administration of any medical agents was defined as a vehicle group.

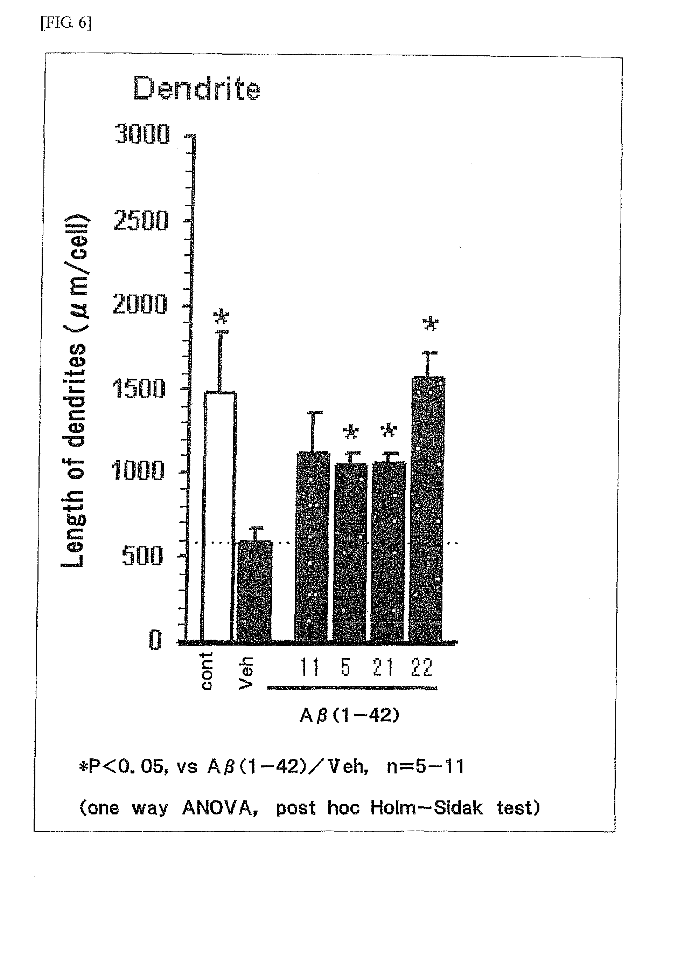

The results are shown in FIG. 6. The vehicle group was ascertained to have the axon atrophy. In contrast, each of the groups treated with Compounds was ascertained to have axon outgrowth effect as remarkably high as that of the control group. In particular, the group treated with Compound 22 was ascertained to have axon outgrowth effect remarkably higher than that of the control group.

The above results reveal that the compound of general formula (1) has the remarkable neuronal cell death inhibition effect and the neurite outgrowth effect, and the compound of general formula (1) is ascertained to be useful for the therapeutic and/or prophylactic agent for neurological disorders.

## Claims

1. A neuronal cell death inhibitor comprising:
a compound of general formula (1) below: 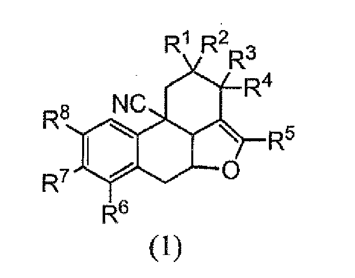 (where R¹ is a hydrogen atom or an optionally substituted hydroxy group; R² is a hydrogen atom; R³ is a hydrogen atom or an optionally substituted hydroxy group; R⁴ is a hydrogen atom or an optionally substituted hydroxy group, R³ together with R⁴ is optionally an oxo group or a group of -O-(CH₂)ₙ-O- (where n is an integer of 2 to 4), or R² together with R⁴ optionally forms an unsaturated bond between carbon atoms, the respective carbon atoms being attached to R² and R⁴; R⁵ is a hydrogen atom or an optionally substituted lower alkyl group; each of R⁶, R⁷, and R⁸ is the same as or different from each other and is a hydrogen atom, an optionally substituted hydroxy group, an optionally substituted alkyl group, an optionally substituted aryl group, or an optionally substituted heteroaryl group); or
a salt thereof.

2. The neuronal cell death inhibitor according to claim 1, wherein each of R¹ and R⁵ is a hydrogen atom.

3. The neuronal cell death inhibitor according to claim 1 or 2, wherein each of R⁶, R⁷, and R⁸ is the same as or different from each other and is a hydrogen atom; a hydroxy group optionally substituted with a sulfonyl group, a silyl group, or a lower alkyl group optionally substituted with a halogen atom; or an aryl group substituted with a halogen

4. A therapeutic and/or prophylactic agent for a neurological disorder, comprising:
a compound of general formula (1) below: 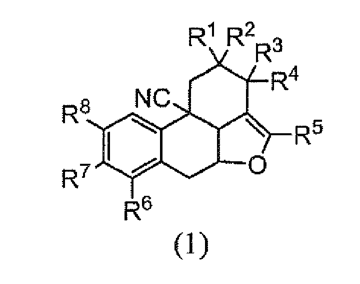 (where R¹ is a hydrogen atom or an optionally substituted hydroxy group; R² is a hydrogen atom; R³ is a hydrogen atom or an optionally substituted hydroxy group; R⁴ is a hydrogen atom or an optionally substituted hydroxy group, R³ together with R⁴ is optionally an oxo group or a group of -O-(CH₂)ₙ-O- (where n is an integer of 2 to 4), or R² together with R⁴ optionally forms an unsaturated bond between carbon atoms, the respective carbon atoms being attached to R² and R⁴; R⁵ is a hydrogen atom or an optionally substituted lower alkyl group; each of R⁶, R⁷, and R⁸ is the same as or different from each other and is a hydrogen atom, an optionally substituted hydroxy group, an optionally substituted alkyl group, an optionally substituted aryl group, or an optionally substituted heteroaryl group); or
a salt thereof.

5. The therapeutic and/or prophylactic agent for a central nervous system disease according to claim 4, wherein each of R¹ and R⁵ is a hydrogen atom.

6. The therapeutic and/or prophylactic agent for a central nervous system disease according to claim 4 or 5, wherein each of R⁶, R⁷, and R⁸ is the same as or different from each other and is a hydrogen atom; a hydroxy group optionally substituted with a sulfonyl group, a silyl group, or a lower alkyl group optionally substituted with a halogen atom; or

7. A use of a compound of general formula (1) below: 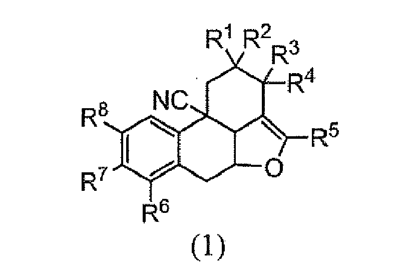 (where R¹ is a hydrogen atom or an optionally substituted hydroxy group; R² is a hydrogen atom; R³ is a hydrogen atom or an optionally substituted hydroxy group; R⁴ is a hydrogen atom or an optionally substituted hydroxy group, R³ together with R⁴ is optionally an oxo group or a group of -O-(CH₂)ₙ-O- (where n is an integer of 2 to 4), or R² together with R⁴ optionally forms an unsaturated bond between carbon atoms, the respective carbon atoms being attached to R² and R⁴; R⁵ is a hydrogen atom or an optionally substituted lower alkyl group; each of R⁶, R⁷, and R⁸ is the same as or different from each other and is a hydrogen atom, an optionally substituted hydroxy group, an optionally substituted alkyl group, an optionally substituted aryl group, or an optionally substituted heteroaryl group)
or a salt thereof; for producing a neuronal cell death inhibitor.

8. The use according to claim 7, wherein each of R¹ and R⁵ is a hydrogen atom.

9. The use according to claim 7 or 8, wherein each of R⁶, R⁷, and R⁸ is the same as or different from each other and is a hydrogen atom; a hydroxy group optionally substituted with a sulfonyl group, a silyl group, or a lower alkyl group optionally substituted with a halogen atom; or an aryl group substituted with a halogen atom.

10. A use of a compound of general formula (1) below: 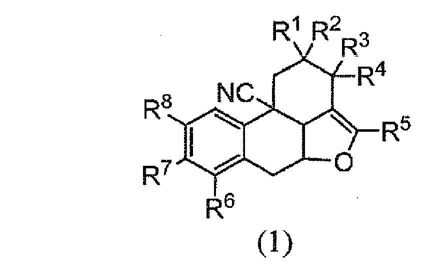 (where R¹ is a hydrogen atom or an optionally substituted hydroxy group; R² is a hydrogen atom; R³ is a hydrogen atom or an optionally substituted hydroxy group; R⁴ is a hydrogen atom or an optionally substituted hydroxy group, R³ together with R⁴ is optionally an oxo group or a group of -O-(CH₂)ₙ-O- (where n is an integer of 2 to 4), or R² together with R⁴ optionally forms an unsaturated bond between carbon atoms, the respective carbon atoms being attached to R² and R⁴; R⁵ is a hydrogen atom or an optionally substituted lower alkyl group; each of R⁶, R⁷, and R⁸ is the same as or different from each other and is a hydrogen atom, an optionally substituted hydroxy group, an optionally substituted alkyl group, an optionally substituted aryl group, or an optionally substituted heteroaryl group)
or a salt thereof; for producing a therapeutic and/or prophylactic agent for a neurological disorder.

11. The use according to claim 7, wherein each of R¹ and R⁵ is a hydrogen atom.

12. The use according to claim 10 or 11, wherein each of R⁶, R⁷, and R⁸ is the same as or different from each other and is a hydrogen atom; a hydroxy group optionally substituted with a sulfonyl group, a silyl group, or a lower alkyl group optionally substituted with a halogen atom; or an aryl group substituted with a halogen atom.

13. A method for inhibiting neuronal cell death **characterized by** comprising administering an effective amount of a compound of general formula (1) below: 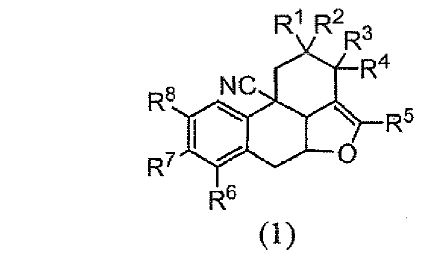 (where R¹ is a hydrogen atom or an optionally substituted hydroxy group; R² is a hydrogen atom; R³ is a hydrogen atom or an optionally substituted hydroxy group; R⁴ is a hydrogen atom or an optionally substituted hydroxy group, R³ together with R⁴ is optionally an oxo group or a group of -O-(CH₂)ₙ-O- (where n is an integer of 2 to 4), or R² together with R⁴ optionally forms an unsaturated bond between carbon atoms, the respective carbon atoms being attached to R² and R⁴; R⁵ is a hydrogen atom or an optionally substituted lower alkyl group; each of R⁶, R⁷, and R⁸ is the same as or different from each other and is a hydrogen atom, an optionally substituted hydroxy group, an optionally substituted alkyl group, an optionally substituted aryl group, or an optionally substituted heteroaryl group) or a salt thereof.

14. The method according to claim 12, wherein each of R¹ and R⁵ is a hydrogen atom.

15. The method according to claim 12 or 13, wherein each of R⁶, R⁷, and R⁸ is the same as or different from each other and is a hydrogen atom; a hydroxy group optionally substituted with a sulfonyl group, a silyl group, or a lower alkyl group optionally substituted with a halogen atom; or an aryl group substituted with a halogen atom.

16. A. method for treating and/or preventing a neurological disorder **characterized by** comprising administering an effective amount of a compound of general formula (1) below: 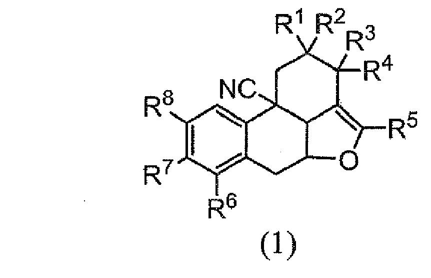 (where R¹ is a hydrogen atom or an optionally substituted hydroxy group; R² is a hydrogen atom; R³ is a hydrogen atom or an optionally substituted hydroxy group; R⁴ is a hydrogen atom or an optionally substituted hydroxy group, R³ together with R⁴ is optionally an oxo group or a group of -O-(CH₂)ₙ-O- (where n is an integer of 2 to 4), or R² together with R⁴ optionally forms an unsaturated bond between carbon atoms, the respective carbon atoms being attached to R² and R⁴; R⁵ is a hydrogen atom or an optionally substituted lower alkyl group; each of R⁶, R⁷, and R⁸ is the same as or different from each other and is a hydrogen atom, an optionally substituted hydroxy group, an optionally substituted alkyl group, an optionally substituted aryl group, or an optionally substituted heteroaryl group) or a salt thereof.

17. The method according to claim 16, wherein each of R¹ and R⁵ is a hydrogen atom.

18. The method according to claim 16 or 17, wherein each of R⁶, R⁷, and R⁸ is the same as or different from each other and is a hydrogen atom; a hydroxy group optionally substituted with a sulfonyl group, a silyl group, or a lower alkyl group optionally substituted with a halogen atom; or an aryl group substituted with a halogen atom.

19. A compound of general formula (1a) below: 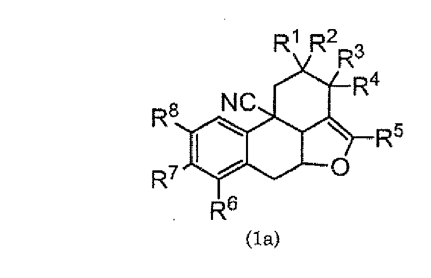 (where R¹ is a hydrogen atom or an optionally substituted hydroxy group; R² is a hydrogen atom; R³ together with R⁴ is a group of -O-(CH₂)ₙ-O- (where n is an integer of 2 to 4); R⁵ is a hydrogen atom or an optionally substituted lower alkyl group; each of R⁶, R⁷, and R⁸ is the same as or different from each other and is a hydrogen atom, an optionally substituted hydroxy group, an optionally substituted alkyl group, an optionally substituted aryl group, or an optionally substituted heteroaryl group, with proviso that cases where each of R⁶ and R⁸ is a hydrogen atom, R⁷ is a hydrogen atom, an optionally substituted hydroxy group, an optionally substituted alkyl group, an optionally substituted aryl group, or an optionally substituted heteroaryl group are excluded) or a salt thereof.

20. The compound or a salt thereof according to claim 19, wherein each of R¹ and R⁵ is a hydrogen atom.

21. The compound or a salt thereof according to claim 19 or 20, wherein each of one or more of R⁶, R⁷ and R⁸ is an optionally substituted hydroxy group and each of the remainder is a hydrogen atom.

## Amended claims

### Amended claims under Art. 19.1 PCT

**1.** 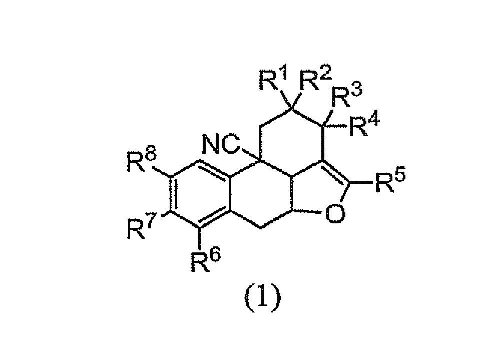 (where R¹ is a hydrogen atom or an optionally substituted hydroxy group; R² is a hydrogen atom; R³ is a hydrogen atom or an optionally substituted hydroxy group; R⁴ is a hydrogen atom or an optionally substituted hydroxy group, R³ together with R⁴ is optionally an oxo group or a group of -O-(CH₂)ₙ-O- (where n is an integer of 2 to 4), or R² together with R⁴ optionally forms an unsaturated bond between carbon atoms, the respective carbon atoms being attached to R² and R⁴; R⁵ is a hydrogen atom or an optionally substituted lower alkyl group; each of R⁶, R⁷, and R⁸ is the same as or different from each other and is a hydrogen atom, an optionally substituted hydroxy group, an optionally substituted alkyl group, an optionally substituted aryl group, or an optionally substituted heteroaryl group).

**17.** The method according to claim 16, wherein each of R¹ and R⁵ is a hydrogen atom.

**18.** The method according to claim 16 or 17, wherein each of R⁶, R⁷, and R⁸ is the same as or different from each other and is a hydrogen atom; a hydroxy group optionally substituted with a sulfonyl group, a silyl group, or a lower alkyl group optionally substituted with a halogen atom; or an aryl group substituted with a halogen atom.

**19.** (Amended) A compound of general formula (1a) below: 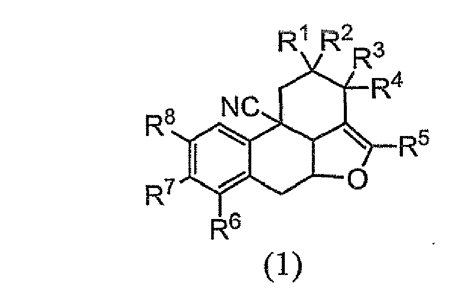 (where R¹ is a hydrogen atom or an optionally substituted hydroxy group; R² is a hydrogen atom; R³ together with R⁴ is a group of -O-(CH₂)ₙ-O- (where n is an integer of 2 to 4); R⁵ is a hydrogen atom or an optionally substituted lower alkyl group; each of R⁶, R⁷, and R⁸ is the same as or different from each other and is a hydrogen atom, a hydroxy group, a lower alkoxy group, a halogeno lower alkoxy group, a phenyl lower alkyloxy group, a halogenophenyl lower alkyloxy group, a lower alkylsulfonyloxy group, a halogeno lower alkylsulfonyloxy group, an optionally substituted alkyl group, an optionally substituted aryl group, or an optionally substituted heteroaryl group, with proviso that cases where each of R⁶ and R⁸ is a hydrogen atom and R⁷ is a hydrogen atom, an optionally substituted hydroxy group, an optionally substituted alkyl group, an optionally substituted aryl group, or an optionally substituted heteroaryl group are excluded) or a salt thereof.

**20.** The compound or a salt thereof according to claim 19, wherein each of R¹ and R⁵ is a hydrogen atom.

**21.** (Amended) The compound or a salt thereof according to claim 19 or 20, wherein each of one or more of R⁶, R⁷, and R⁸ is a hydroxy group, a lower alkoxy group, a halogeno lower alkoxy group, a phenyl lower alkyloxy group, a halogenophenyl lower alkyloxy group, a lower alkylsulfonyloxy group, or a halogeno lower alkylsulfonyloxy group, and each of the remainder is a hydrogen atom.
